# EUROPEAN PATENT SPECIFICATION

(11) **EP 0 663 008 B1**
(45) Date of publication and mention of the grant of the patent: **02.01.2004**
(21) Application number: 93921048.0
(22) Date of filing: 04.10.1993
(51) Int. Cl.: C12N 15/13, C12N 15/62, C12N 15/48, C12N 15/19, A61K 31/70, A61K 39/395

(54) **IMPROVEMENTS IN OR RELATING TO IMMUNE RESPONSE MODIFICATION**
VERBESSERUNGEN IN ODER BEZOGEN AUF MODIFIKATIONEN DER IMMUNANTWORT
AMELIORATIONS RELATIVES A LA MODIFICATION DE REPONSE IMMUNITAIRE

(30) Priority: 02.10.1992 GB 9220808
(43) Date of publication of application: 19.07.1995
(73) Proprietor: Cancer Research Technology Limited, London WC2A 3PX (GB)
(72) Inventor: HAWKINS, Robert Edward, Cambridge CB3 0RU (GB); RUSSELL, Stephen James, Cambridge CB2 5ER (GB); STEVENSON, Freda Katherine, Bassett, Southampton SO1 7AD (GB); WINTER, Gregory Paul, Cambridge CB1 4UT (GB)
(74) Representative: Cripps, Joanna Elizabeth
(86) International application number: PCT/GB1993/002054
(87) International publication number: WO 1994/008008

(56) References cited:
- WO-A-92/01047
- EUR. J. BIOCHEM. vol. 22 , March 1992 , SPRINGER VERLAG, BERLIN, BRD; pages 867 - 870 R.E. HAWKINS AND G. WINTER 'Cell selection strategies for making antibodies from variable gene libaries: trapping the memory pool' cited in the application
- JOURNAL OF IMMUNOLOGY vol. 139, no. 8 , 15 October 1987 , WILIAMS AND WILKINS, BALTIMORE, US; pages 2825 - 2833 M.J. CAMPBELL ET AL. 'Idiotype vaccination against murine B cell lymphoma' cited in the application
- NUCL. ACIDS RES. vol. 20, no. 15 , 11 August 1992 , IRL PRESS, OXFORD, ENGLAND; pages 3831 - 3837 M.J. EMBLETON ET AL. 'In-cell PCR from mRNA: amplifying and linking the rearranged immunoglobulin heavy and light chain V-genes within single cells' cited in the application
- BIO/TECHNOLOGY vol. 10, no. 7 , July 1992 , NATURE AMERICA, INC., NEW YORK, US; pages 779 - 783 J.D. MARKS ET AL. 'By-passing immunization: Building high affinity human antibodies by chain shuffling'
- BIO/TECHNOLOGY vol. 9, no. 2 , February 1991 , NATURE AMERICA, INC., NEW YORK, US; pages 165 - 169 G.T. DAVIS ET AL. 'single chain antibody ENCODING genes: One-step construction and expression in eucaryotic cells' cited in the application
- INTERN. REV. IMMUNOL. vol. 4 , 1989 , HARWOOD ACADEMIC PUBLISHERS, NEW YORK,US; pages 271 - 310 A.J.T. GEORGE AND F.K. STEVENSON 'prospects for the treatment of B cell tumors using idiotypic vaccination' cited in the application
- NATURE vol. 356 , 12 March 1992 , MACMILLAN JOURNALS LTD., LONDON, UK; pages 152 - 154 D.-C. TANG ET AL. 'Genetic immunization is a simple method for elicting an immune response' cited in the application
- FEBS LETT. vol. 288, no. 1,2 , August 1991 , ELSEVIER PUBLISHERS, AMSTERDAM, NL; pages 138 - 142 S. HINUMA ET AL. 'A novel strategy for converting recombinant viral protein into high immunogenic antigen'
- J. MOL. BIOL. vol. 226 , 1992 , ACADEMIC PRESS LIMITED, LONDON, UK; pages 889 - 896 R.E. HAWKINS ET AL. 'Selection od phage antibodies by binding affinity' cited in the application
- NATURE vol. 362 , 22 April 1993 , MACMILLAN JOURNALS LTD., LONDON, UK; pages 755 - 758 M.-H. TAO AND R. LEVY 'Idiotype/granulocyte-macrophage colony-stimulating factor fusion protein as a vaccine for B-cell lymphoma' cited in the application

## Description

### Field of the Invention

This invention relates to a method of modifying the immune response of an individual and to novel compositions for performing the method of the invention and methods of making said compositions.

### Background of the Invention

There have been several reports describing the introduction of DNA, packaged in retroviral vectors, into mammalian hosts. More recently Wolff et al. (1990 Science 297, 1465-1468) described experiments in which RNA and DNA expression vectors (carrying reporter genes such as chloramphenicol acetyltransferase [CAT], luciferase and β-galactosidase) were injected into mouse skeletal muscle, without the use of a viral packaging vector. Various assays showed that the reporter genes were expressed in some of the muscle cells.

Subsequently, Felgner & Rhodes (1991 Nature 349, 351-352) reviewed the results of various experiments which involved the use of injected DNA (both packaged and 'naked') to direct the expression of foreign genes, and suggested the idea of using injected DNA for the purpose of immune regulation. In particular they stated that "Some of these concepts have been tested by using a plasmid containing the gene for a secreted form of the human immunodeficiency virus gp120 protein and driven by the cytomegalovirus [CMV] promoter". They found that a single injection of the DNA induced a high IgG antibody titre against the foreign protein.

In 1992 Tang et al. (Nature 356, 152-154) further developed the idea of "genetic immunisation". These workers used gold microparticles, coated with plasmid DNA, to introduce into mice a gene coding for the expression of human growth hormone (hGH) under the control of either the human β-actin or the CMV promoter. The mice were later shown to develop a serum antibody response to hGH.

A.J.T. George and F.K. Stevenson (Intern. Rev. Immund. 4, 1989, pp 271-310) teaches the use of an idiotypic antibody derived from a tumour cell for use in vaccinating the tumour bearing host. They illustrate the success of using such self proteins for immunizations.

D. Tang et al (Nature Vol 356, 12 March 1992) discloses the use of a foreign antigen (hGH) as a DNA vaccination. They report that the gene encoding a protein can be introduced directly into a host, and that the encoded protein provokes an immune response.

In summary, it has been shown that it is possible to use genetic immunisation to generate in mice an immune response to strongly immunogenic foreign proteins. It has not hitherto been suggested or thought possible that such methods could be used to regulate the immune response to substantially non-immunogenic "self" or "altered self" polypeptides.

### Summary of the Invention

In a first aspect the invention provides an immunomodulatory composition to modulate the immune response against a tumour associated antigen, said composition comprising a nucleic acid sequence directing the expression of said tumour associated antigen in a form which interacts with the immune system of the individual.

Preferably the nucleic acid sequence is one which has been extracellularly cloned.

Such a tumour associated antigen could be, for example, the idiotypic determinant of an immunoglobulin (expressed on the surface of a B cell lymphoma).

A tumour associated antigen is derived in an individual from a self polypeptide by processes occurring in vivo which alter the nucleic acid sequence encoding said self polypeptide. Such processes would include, for example, deletions, additions, substitutions and translocations.

Preferably the nucleic acid sequence is cloned from samples obtained from the individual to whom it is delivered.

Preferably the nucleic acid sequence is delivered in unencapsidated form (i.e. not enclosed within a viral particle or other package). The nucleic acid may, however, be associated with the external surface of a package or particle (e.g. a liposome).

Following delivery to the individual, the tumour associated antigen may be expressed in isolation. More preferably however, the tumour associated antigen is co-expressed, or expressed as a fusion, with a further immunomodulatory polypeptide.

It is thus a preferred feature of the present invention that the immunomodulatory composition further comprises a second nucleic acid sequence which directs the expression of a further immunomodulatory polypeptide for the purpose of further modulating the immune response to the tumour associated antigen. This second nucleic acid sequence may be comprised on the same nucleic acid molecule as the first nucleic acid sequence, or may be present on a second nucleic acid molecule.

The further immunomodulatory polypeptide may be, for example, a cytokine or a foreign polypeptide (e.g. a viral envelope protein).

Where both the first and second nucleic acid sequences are present on the same nucleic acid molecule, the sequences may be so arranged as to provide for the expression of a fusion polypeptide comprising both the tumour associated antigen and the further immunomodulatory polypeptide.

Where the first and second nucleic acid molecules are present on different molecules, the arrangement is preferably such that the tumour associated antigen are co-expressed when introduced into the individual. Further, the arrangement may be such that the tumour associated antigen and the further immunomodulatory polypeptide may associate (indirectly or directly) when expressed in the same individual. Typically the nucleic acid sequence is a deoxyribonucleic acid sequence.

It will be apparent to those skilled in the art that the immunomodulatory composition of the present invention could be used to either enhance or suppress the immune response to a particular tumour associated antigen. It will clearly be desirable to enhance the immune response to the antigen so as to halt or reduce the growth of the tumour. Enhancement of the immune response to the following tumour associated antigen may be desirable: idiotypic determinants on the immunoglobulin expressed on the surface of B cell malignancies; idiotypic determinants of T cell receptors (TCRs) expressed on the suface of T cell malignancies; mutated oncogenes or other self polypeptides expressed on the surface of tumours; and oncofoetal antigens.

However one can also envisage situations where one would wish to reduce the immune response to a particular self or altered self polypeptide. For example, self polypeptides can be the subject of an inappropriate immune response resulting in a damaging autoimmune disease (e.g. rheumatoid arthritis, multiple sclerosis, diabetes). Alternatively, patients can mount an immune response to therapeutic proteins (e.g. antibodies, insulin given to diabetics, factor VIII given to haemophiliacs) which therefore reduces the efficacy of the treatment. A further possibility would be the suppression of immune responses to MHC antigens, thereby reducing problems with transplant rejection, allowing the possibility of transplanting across MHC incompatibility barriers. The cytokine interleukin-10 (IL-10) has been shown to exert an immunosuppressive effect, thus co-expressing a self or altered self polypeptide with IL-10 in a patient according to the method of the invention could be a desirable approach.

In a second aspect, the invention provides a method of making an immunomodulatory composition to modulate the immune response against a tumour associated antigen, comprising: identifying the nucleic acid sequence encoding the tumour associated antigen by analysis of a suitable sample from a patient and extracellularly cloning therefrom; inserting said nucleic acid sequence into a suitable vector and administering said vector to a patient so as to cause expression of the disease marker in a form which interacts with the immune system.

Typically the nucleic acid encoding said tumour associated antigen is cloned from the patient by means of PCR.

Typically, the tumour associated antigen will be an idiotypic determinant from an immunoglobulin expressed on the surface of a B cell malignancy or an idiotypic determinant from a T cell receptor expressed on the surface of a T cell malignancy. Alternatively the tumour associated antigen is an oncofoetal antigen.

In a third aspect the invention provides a composition for delivery into living cells in vivo for modifying the immune response of an individual to a tumour associated antigen, comprising a nucleic acid sequence directing the expression of a tumour associated antigen in a form which interacts with the immune system of the individual.

The invention further provides the use of an immunomodulatory composition according to the invention in the preparation of a medicament for modifying the immune response of an individual, wherein said modification comprise delivery into living cells in vivo of the nucleic acid sequence directing expression of said tumour anociated antigen in a form which interacts with the immune system of the individuals.

In a particular embodiment this invention provides a vaccine nucleic acid which can be used to elicit an immune response against transformed human lymphocytes displaying an idiotypic marker encoding proteins comprising the heavy and light chain variable regions of an idiotypic antibody displayed on surface of a malignant human B-cell.

The invention will be further described by way of example and by reference to the drawings, of which:
Figure 1 is a schematic representation of the method of PCR assembly of DNA expressing scFv;
Figure 2 shows the sequence of the vectors used to express and purify scFv idiotypic immunoglobulin;
Figure 3 is a schematic representation of the method used to produce plasmid pNipenv;
Figure 4 is a schematic representation of the plasmid constructs pNipenv, pSV2 Nipenv, pSV2 Nip stop env, and pSV2 BCL env;
Figure 5 shows the sequence of a HindIII - XbaI fragment of the vector pVAC1;
Figure 6 is a graph showing the results of an immunisation experiment;
Figure 7 shows the entire sequence of the vector pVAC1;
Figure 8 shows a pVAC1 restriction map;
Figure 9 shows a schematic representation of the main features of pVAC1;
Figure 10 is a graph showing the results of an immunisation experiment; and
Figure 11 (a,b) shows the results of FACS analysis.

The idiotypic determinants of surface immunoglobulins expressed on certain types of B cell Non Hodgkin's Lymphoma (NHL) are unique tumour-specific antigens. As such, they should be suitable targets for various immunotherapeutic anti-lymphoma strategies. Murine monoclonal antibodies (MAbs) raised against idiotypic determinants on B cell NHLs have given limited benefit in human therapeutic trials. Partial and complete responses have been observed, but the murine MAbs tend to recruit human effector functions inefficiently and are themselves the target of a human anti-mouse antibody response. Also, outgrowth of surface Ig negative lymphoma cells has been observed following therapy. In light of these limitations, coupled with the cost and inconvenience of generating MAbs for individual patients, the approach has not been widely adopted. However, it is clear that anti-idiotypic antibodies do have therapeutic potential in B cell NHL.

One alternative to passive anti-idiotypic serotherapy is active immunisation which aims to break tolerance and induce a strong anti-idiotypic antibody response in the patient. An additional advantage of this approach is that it also has the potential to stimulate T cell mediated immune responses against the lymphoma. The problem is how best to present the antigen (the idiotypic antibody) to break tolerance and stimulate an effective anti-lymphoma immune response. Efforts to stimulate tumour immunity using modified tumour cell vaccines have met with limited success. Specific vaccines based on using idiotypic immunoglobulin chemically coupled to highly immunogenic carrier proteins have proved more successful in animal models providing both protection and therapeutic effect (George and Stevenson, 1989 Intern. Rev. Immunol. 4, 271-310).

For lymphomas, which secrete little immunoglobulin, making the idiotype is a major problem. The present inventors have explored various strategies to develop simple, effective vaccines that can be made on an individual patient basis using PCR and DNA sequencing methods to identify the genes of the idiotypic immunoglobulin.

In a particular embodiment the invention provides a nucleic acid vaccine that is used to elicit an immune response against human lymphocytes displaying a B or T-cell, antibody or TCR, idiotypic marker, and in which the nucleic acid encodes polypeptides comprising both variable regions (VH/VL; Va/Vb; Vg/Vd).

The idiotypic determinants expressed on the cell surface immunoglobulin (Ig) of B-cell lymphomas can act as tumour-associated antigens (for review see George and Stevenson, 1989). Likewise cell surface TCR could also act as a tumour associated antigen in T-cell lymphomas (Janson et al., 1989 Cancer Immunol. Immunother. 28, 225-232). As such they present an attractive target for therapy, notably for the administration of passive anti-idiotypic antibody to patients (Miller et al., 1982 New Engl. J. Med. 306, 517-522). Although somatic mutation of the target malignant B-cells can result in escape of the target from the anti-idiotypic antibody (Levy et al., 1987 J. Immunol. Rev. 96, 43-; Bahler and Levy, 1992 PNAS 89, 6770-6774) the complete loss of immunoglobulin expression is rare (Meeker, et al., 1985 New Engl. J. Med. 312, 1658-1665; Zelentz et al., 1990 Ann. Oncol. 2, 115-122). Another approach is the use of the idiotypic determinants for active immunotherapy, that is, to immunise the tumour bearing host with idiotypic Ig from the tumour cells, thereby generating an autologous anti-idiotypic response (see George and Stevenson, 1989). Since the response will be polyclonal, it is more difficult for the target B-cell to escape selection, and furthermore, the response will be present on a continuing basis, and so might be able to control residual disease. In fact, active immunisation with idiotypic Ig prior to tumour challenge, has proved effective in suppressing model B-cell tumours (Stevenson and Gordon, 1983 J. Immunol. 130, 970-973; George et al., 1987 J. Immunol. 138, 628-634; Campbell et al., 1987 J. Immunol. 139, 2825-2833) in animals and to treat animals bearing incipient tumour (George et al., 1988 J. Immunol. 141, 2168-2174). Furthermore idiotypic immunisation with human Ig isolated from patients with lymphoma has been associated with sustained tumour regression (Kwak et al., 1992 New Engl. J. Med. 327, 1209-1215). However it is difficult and time consuming to isolate the idiotypic human antibody as for non-Hodgkin's lymphoma, although it is on the surface of the lymphocytes, little immunoglobulin is secreted. To make sufficient idiotypic antibody for immunisation heterohybridomas must be prepared by fusion with mouse cell lines and the antibody then purified (Carroll et al., 1985 J. Immunol. Methods 89, 61-67). The yield is frequently low and it must be subsequently confirmed that the fusion derives from the human B-cell tumour.

In a particular embodiment the invention entails the isolation of the V-genes from the tumour B-cells to express the idiotope (and paratope) of the tumour antibody. Thus starting with a sample of B-cells from the patient, the rearranged V-genes of both heavy and light chains are amplified using the polymerase chain reaction and "universal" primers (Orlandi et al., 1989 PNAS 86, 3833-3837; Marks et al., 1991 J. Mol. Biol. 222, 581-597; see also Table 1 Seq. ID Nos. 1-48). The amplified V-genes are cloned and then sequenced (Sanger et al., 1977 PNAS 74, 5463-5467). Those from the malignant B-cells are identified as repeated VH and VL gene sequences. In several patients, and for both heavy and light chains, it was possible to identify a common repeated sequence. These sequences were subsequently confirmed by sequencing the genes from heterohybridomas in the case of three patients. The combination of the heavy and the light chains identifies the idiotope of the tumour (Example 1). In principle, it would also be possible to amplify and link the rearranged V-genes within the same cell (Embleton et al., 1992 Nucl. Acids Res. 20, 3831-3837) to identify a major combination of linked heavy and light chain sequences that identify the idiotope but here the VH and VL are separately identified and then linked by PCR assembly. The VH and VL genes are cloned into vectors for the expression of both V-domains as a functional antibody fragment, for example as a linked single chain Fv fragment (see below).

In previous work, in a mouse model for lymphoma, the V-genes of the tumour idiotope were cloned and expressed as light and heavy chain fusion proteins in bacteria. The separate chains were then used as immunogens. However the separate chains were denatured, and in any case were not co-expressed to provide the paratope of the antibody. Indeed the authors suggested that "future work on peptides with fixed configurations similar to epitopes present in the native protein may prove useful, as may the co-expression of both VH and VL genes in bacteria to produce a recombinant Fv protein" (Campbell et al., 1987, cited above). However the authors did not teach how to isolate the V-genes of the idiotope. Nor did the authors teach how to combine the recombinant Fv fragments into a vaccine. Ideally a vaccine should be capable of stimulating antigen-specific B cells, cytotoxic T lymphocytes (CTLs) and helper T cells. B cell stimulation requires that the target antigen should bind with sufficiently high affinity to specific antigen receptors (surface Ig) on the B-cell surface. Certain multivalent antigens can stimulate B cell proliferation directly but more often, and to provide an effective anamnestic response, there is a requirement for additional signals provided by helper T cells (see below).

The T cell receptors (TCRs) of CTLs recognise specific MHC class 1-associated peptides displayed at the target cell surface. Such peptides are generally derived by processing of larger polypeptides or proteins manufactured within the target cell. Thus, for efficient CTL stimulation the target antigen should be synthesised intracellularly in MHC class I-expressing cells. The level of expression should be high enough to generate sufficient peptide to displace those self peptides which are normally bound (possibly with higher affinity) in the MHC peptide-binding groove (Ohno, 1992 PNAS 89, 4643-4647). Similar to antigen-specific B cells, for proliferation and increased cytotoxic capability, CTLs require additional signals (in the form of cytokines) following antigen recognition and these are provided by helper T cells.

T cell help is required for optimal B cell and CTL responses. CD4-positive helper T cells interact (via their unique TCRs) with specific cell surface MHC class II-associated peptides and such peptides are generally derived by proteolytic cleavage of protein antigens internalised by specialised antigen presenting cells (APCs). Macrophages, dendritic cells and B lymphocytes are amongst the cells which can present antigen in this way. Thus, B lymphocytes internalise and process antigen bound to their surface Ig and subsequently present MHC class II-associated derivative peptides. CD4-positive T helper cells recognising the surface peptide can then release various immunostimulatory cytokines and stimulate further B cell activation, proliferation and antibody production. Similarly, macrophages present at the site of a local inflammatory response can process phagocytosed antigen and stimulate cytokine release by T helper cells, leading to enhanced activation, proliferation and cytotoxicity of locally resident CTLs.

The vaccine antigen should therefore ideally 1) be synthesised intracellularly by MHC class I-positive host cells, 2) give rise to peptides which, when displayed by host cell class I MHC can stimulate a subset of host CTLs via their TCRs, 3) give rise to peptides which, when displayed by host cell class II MHC can stimulate a subset of host helper T cells via their TCRs, 4) be internalised and processed by host APCs including both macrophages and antigen-specific B cells, 5) be available in its native form for interaction with host B lymphocytes.

In a further specific embodiment, the invention provides for the expression of the rearranged VH and VL genes of the idiotope of the tumour antibody within mammalian cells, allowing the production of peptides for display on the cell surface in combination with host MHC, and for display (or secretion) of the paratope (as a folded antibody fragment), to trigger the production of anti-idiotypic antibodies. The antibody fragments could be introduced into mammalian cells by infection with a recombinant virus encoding the antibody fragments. In principle, the antibody fragments could be provided with a signal sequence for their secretion or display on the surface of the infected (transfected) cell. Alternatively the fragments could be linked to another protein that is displayed on the surface of the cell, for example a viral coat protein, as described in Example 2. The antibody fragments (as single chain Fv fragments) are displayed in a functional form attached to the coat protein of a virus, indicating that they are also folded and in a native form on the surface of an infected cell (Russell et al., 1993 Nucl. Acids Res. 21, 1081-1085). The antibody fragments could also be introduced into mammalian cells using nucleic acid encoding the antibody fragments. For example, a gene encoding a fusion protein between a viral coat protein (as above) and the antibody fragments was used to immunise mice by direct injection (subcutaneous or intramuscularly, see Example 3).

Although the examples (below) are primarily concerned with the isolation of idiotypic markers in lymphocyte maligancies, it should also be possible to immunise against B-and T-cell idiotopes involved in auto-immune disease in the same way, provided it is possible to identify the V-gene pairings. Certainly monoclonal antibodies have been made with reactivities against self-antigens from the B-cells of auto-immune patients. Presumbly linking combinations of heavy and light chains together within B-cells (Embleton et al., 1992) followed by cloning into phage (McCaffeny et al., 1992 Nature 348, 552-554), and the subsequent selection for phages with self-specificities will facilitate the identification of antibodies involved and allow their use for anti-idiotypic therapy. For other autoimmune diseases certain TCR families are known to be over-represented (Marguerie et al., 1992 Immunol. Today 13, 336-338) and thus in cell PCR assembly followed by sequencing should allow identification of the receptors involved and their subsequent use for anti(TCR)-idiotypic immunisation.

### Examples

### Example 1 - Indentification of V-genes from biopsies of B-cell lymphoma.

### Preparation of biopsy material.

Biopsy specimens were obtained from five patients with pathologically confirmed Follicular Lymphoma. They were obtained during routine diagnostic procedures. The light chains were identified as kappa or lambda by immunohistochemistry. As non-malignant controls, a small bowel lymph node from from a patient with Crohn's disease and a sample of spleen from a patient undergoing splenectomy were obtained. Biopsy material was prepared as a single cell suspension and the cells subsequently frozen and stored at -70°C.

### Preparation of DNA for PCR.

For PCR the DNA was prepared using a simple proteinase K/Tween 20 lysis method (Innis et al., 1990 PCR Protocols: A Guide to Methods and Applications; Academic Press Inc., p147). Briefly the cells were pelleted by centrifugation for 20 seconds at 13,000 rpm in a microcentrifuge. The cells were then washed twice with 1 ml PBS before resuspending at approximately 10⁶/ml in K-buffer (10mM Tris.Cl (pH 8.3), 50 mM KCl, 1.5 mM MgCl₂, 0.5% Tween 20, 100mg/ml proteinase K) and incubated at 56°C for 60 minutes to lyse the cells and release DNA. The proteinase K was then inactivated by incubation for 30 minutes at 95°C. DNA thus released was used directly in the PCR reactions or stored for subsequent use at -20°C.

### PCR primers

PCR primers were designed to amplify re-arranged heavy chain kappa and lambda light chain genes. The 5' primers are based on framework 1 of the V-genes. The VH and Vk primers are similar to those described by Marks et al. (1991). However, for amplification from genomic DNA (as opposed to cDNA) the product was found to be cleaner if primers shortened by one base at the 3' end were used (data not shown). In addition, the number of primers used was reduced by combining similar primers as one consensus primer. With the exception of one change in the JH primers, to introduce a common BstEII site, changes were not made to introduce restriction sites. Limited DNA sequence information was available on which to base the Vλ primers but primers were made to Vλ1, Vλ2, Vλ3 and Vλ4 families from the available sequence data (Songsivilai, et al. 1990 Eur. J. Immunol. 20, 2661-2666; Alexandre et al., 1989 Nucl. Acids Res. 17, 3975; Bernard et al. 1990 Nucl. Acids Res. 18, 7139; Chuchana et al., 1990 Eur J. Immunol. 20, 1317-1325). Other families are known to exist (Chuchana et al., 1990) but there were no nucleotide sequence data available and so primers were not made. J-region primers were made to be complementary to the genomic sequence of the germline J-regions for heavy chain (Ravetch et al., 1981 Cell 27, 583-591), kappa chain (Hieter et al., 1982 J. Biol. Chem. 257, 1516-1522) and lambda chain (Udey and Blomberg 1987 Immunogenetics 25, 63-70; Dariavach 1987 PNAS 84, 9074-9078; Bauer and Blomberg 1991 J. Immunol. 146, 2813-2820; Combriato and Klobeck, 1991 Eur. J. Immunol. 21, 1513-1522; Frippiat, 1990 Nucl. Acids Res. 18, 7134). The Jλ genes combine with their respective Cλ genes and thus since Cλ4, Cλ5 (Dariavach, 1987) and probably Cλ6 (Bauer and Blomberg, 1991; Combriato and Klobeck, 1991) are pseudogenes they should not appear as expressed protein. As a result primers to these Jλ genes were not made. By combining two J region primers, in all three J1 primers were made Jλ1,Jλ2/3, Jλ7. Table 1 gives a full list of the primary PCR primers.

### PCR amplification of rearranged immunoglobulin variable regions

The V-gene family and J-region primers were used as equimolar mixes of the individual primers shown in Table 1. VHBACK and JHFOR mixes were used for the heavy chain PCR reaction. Similar mixes were used for kappa or lambda chain PCR amplification. PCR amplification was performed in 50µl volume using Hybaid Thermal Reactor (Hybaid). Reaction mixtures containing 20pmol of each primer mix, 250µM dNTPs (Pharmacia, Uppsala, Sweden) in 1 x PCR buffer (Promega, 10mM Tris.Cl[pH8.8], 50 mM KCl, 1.5 mM MgCl₂, 0.1% Triton X-100). To minimise any risk of contamination extensive precautions were taken. The mixes were set up in a laminar flow hood in a room designated specifically for setting up PCR reactions. The samples were then UV treated for 5 minutes in a UV oven (Amplirad, Genetic Research, Dunmow, UK). The template (5 µl) was then added, the reaction mix was then overlaid with mineral oil (Sigma) and the sample heated to 94°C for 5 minutes. At this stage Taq DNA polymerase (Promega), 2.5 units, was added. Amplification was performed using 35 cycles, 94°C 1 min, 65°C, 1 min annealing; 72°C, 1 min elongation. Amplified variable regions were analysed on a 1.5% LMP agarose/TAE gel and visualised with ethidium bromide. The band of size 320/350 base pairs was excised and purified using a GENECLEAN II kit (Bio101) according to the manufacturers instructions. At least two independent PCR amplifications of V regions were performed from the sample of every patient and the PCR of lymph node DNA was performed before the corresponding PCR from the heterohybridomas.

### Cloning and sequencing of PCR products

The T-vector cloning system described by Marchuk (Marchuk et al, 1991 Nucl. Acids Res. 19, 1154) was used. In brief, the vector was prepared from pBluescript II KS+ (Stratagene) by digestion with EcoRV (from NBL) to produce blunt ends and then treatment with Taq DNA polymerase (Promega) in PCR buffer (Promega) containing 2mM dTTP at 70°C for 2 hours. The purified V-gene PCR product was ligated into the T vector and transformed into competent E. coli - strain TG1 (Gibson, 1984 Ph.D. thesis, University of Cambridge, United Kingdom). Recombinant clones were identified by blue/white selection using isopropyl-β-thiogalactosidepyranoside (IPTG, Sigma). Random recombinant clones were picked and ssDNA prepared after superinfection with helper phage (M13K07, Stratagene) (Vieira and Messing, 1987 Methods Enzymol. 153, 3-11). The clones were sequenced by the dideoxy method (Sanger et al., 1977) using T7 DNA polymerase (Sequenase, USB, Cleveland, USA). A number of clones from each patient were sequenced and the sequences compared.

### Assembly of Tumour V-genes as scFv

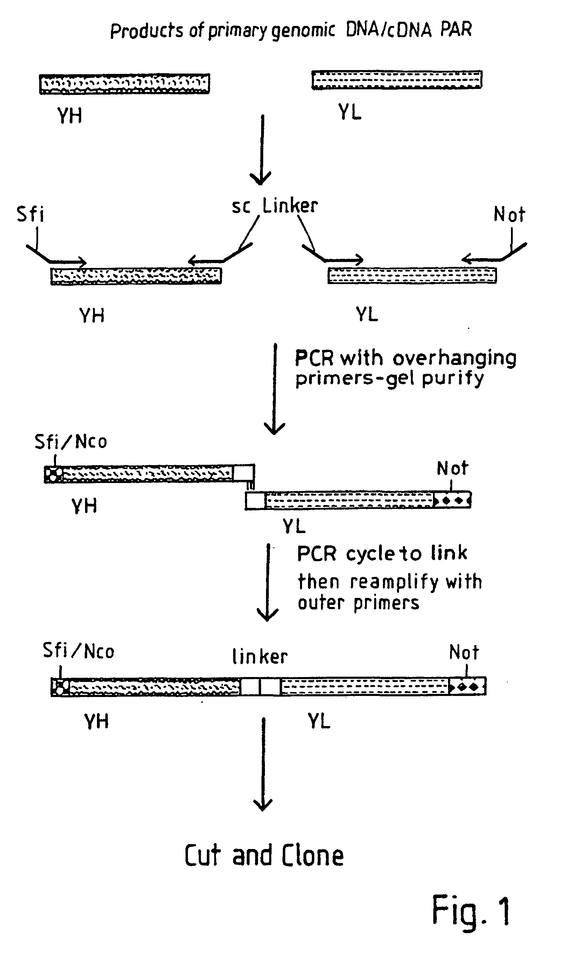

The assembly method, illustrated in figure 1, is based on that described by Davis et al., (1991 Bio/Technology 9, 165-169). The assembly process uses a second set of primers. The VHSfiBAK primers encode SfiI cloning site and also hybridise to the original set of VHBAK primers. The scJHFOR and scVk/Vλ BAK primers hybridise to their respective initial primers but also encode the sc linker to allow production of a single chain Fv (scFv) (Huston et al., 1988 PNAS 85, 5879-5883). The NotJk/IFOR primers hybridise to their respective initial primers but also include the NotI restrction site. These primers are also summarised in Table 1. The assembly is carried out in two stages. First the V-genes (heavy and light chains) were amplified from the sequencing template using the new set of oligonucleotides. The PCR mixture was made up as above but the primers used were only the relevant V-gene family and J-region primers identified by previous sequencing. Template was 100ng of ssDNA sequencing template. The PCR conditions were 94°C for 1 minute, 50°C for 1 minute, 74°C for 1 minute with 10 cycles of amplification. At the completion of the PCR the further dNTPs were added (5 µl of 2.5 mM stock solution) with Klenow polymerase (Boehringer, 2.5 units) and then incubated at 20°C for 15 minutes to produce blunt ends. Following this step the product was gel purified as above and resuspended in 25 µl water. Then 5 µl from the heavy and 5 µl from the light chain product were used in the assembly. For this process the PCR reaction was carried out in two steps. Initially no primers were added and the following cycles were used : 94°C for 1 minute, 50°C for 1 minute, 74°C for 1 minute for 7 cycles to join the heavy and light chains. During the secondary PCR described above the heavy chain and light chain are tagged with primers encoding the single chain linker. This tag contains 15 nucleotides on each of the heavy and light chains complementary to each other and thus allows them to anneal to each other. During the extension reaction full length joined scFv molecules are formed. At the end of these 7 cycles the temperature is held at 94°C for 3 minutes and the relevant outer primers (SfiVHBACK/NotJFOR) added for the "pull-through" amplification. This amplification consists of 10 cycles : 94°C for 1 minute, 74°C for 2 minutes an serves to amplify the small amount of linked product formed.

### Cloning for Expression, and Expression and Purification of scFv

After assembly the scFv was digested with SfiI/NotI as described (Marks et. al., 1992) and cloned into a scFv expression vector (Hawkins et al., 1992 J. Mol. Biol. 226, 889-896) based on pUC119 (Vieira and Messing, 1987). A new expression vector, pRH2, which has the Myc Tag replaced by a hexahistidine tag was made to allow purification using metal affinity chromatography. This was made by inverted PCR site directed mutagenesis (Hemsley et al., 1989 Nucl. Acids Res. 17, 6545-6551). The vectors are shown in Figure (2 Seq. ID Nos. 59-62).

To check for expression of full length scFv individual colonies were picked and grown for four hours with constant shaking in 1 ml 2xTY/ 0.1 % Glucose/100mg/ml Ampicillin at 30°C. At that stage IPTG was added to a final concentation of 1 mM and shaking continued for 18 hours. Supernatant was harvested by centrifugation at 13,000 rpm in a microcentrifuge for 5 minutes. The bacterial pellet was frozen at -20°C for preparation of plasmid DNA and the supernatant was analysed by Western blotting using the 9E10 anti-Myc antibody (Ward et al., 1989 Nature 341, 544-546). Plasmid DNA was then prepared from the bacterial pellet of colonies thus shown to express full length scFv. From this plasmid preparation the scFv was subcloned as an SfiI/NotI fragment into pRH2. One litre cultures of bacteria were grown with constant shaking in 2 litre flasks containing 2xTY/0.1% Glucose/100mg/ml Ampicillin at 30°C to an A600nm of 0.9. At this stage IPTG was added to a final concentration of 1 mM and the incubation continued for a further 4 hours. The bacteria were then pelleted by centrifugation and the periplasmic fraction was prepared as described by Skerra et al., (1991 Bio/Technology 9, 273-278).

The scFv antibody fragment was purified from the periplasmic fraction utilising the hexahistidine tag. The method is based on that described by Skerra et al., (Skerra et al., cited above) but it was found that the use of six histidines and nickel rather than five histidines and zinc was preferrable (data not shown). The periplasmic preparation from a 1 litre culture was loaded onto a 1 ml column of Chelating Sepharose Fast Flow (Pharmacia) previously coupled with nickel ions according to the manufacturers instructions. The column was then washed with 10 ml of PBS/1M NaCl (pH 7.2) followed by 5 ml PBS/1M NaCl/75 mM Imidazole (pH7.2). The retained scFv was then eluted with 5 ml PBS/1M NaCl/300 mM Imidazole (pH 7.2) and collected as 1 ml fractions. The peak protein fractions were identified by determining the A280nm and these were then dialysed against PBS before analysis by SDS-PAGE.

### PCR, cloning and sequencing of V-genes from Follicular Lymphoma and normal lymph node

The PCR amplification from the DNA of biopsy specimens was successful in all cases apart from the lambda light chain from patient number 5. A number of clones from each patient were sequenced. Analysis of the sequences derived from the reactive lymph node and from the normal spleen revealed that there were no repeated sequences. From each of the tumour bearing lymph nodes there were single repeated sequences. A summary of the sequencing results are shown in Table 2. Amongst the repeated sequences there were up to two base changes which were presumed to result from PCR errors. Nevertheless a consensus sequence was readily apparent and in each case there were clones with this consensus sequence. To confirm the sequence a second independent amplification was performed and further V-genes sequenced. The same consensus sequence was identified. The repeated V-gene sequences suggest clonal expansion and thus identifies the tumour V-gene. For three of the five tumour biopsies analysed here a heterohybridoma was available. PCR amplification, cloning and sequencing confirmed the sequence identified direct from the lymph node.

The absolute percentage of the tumour derived V-gene varied and there are several reasons for this. First, the biopsies vary in the degree of tumour infiltration (although in all cases examined here malignant B-cells comprise > 50% of the total cells present). Second, the primers will vary in the efficiency with which they amplify any particular gene - in extreme cases as with the lambda light chain in patient 4 a chain may not amplify at all. Third, some pseudogenes can be amplified by these primers and this may reduce the overall percentage of tumour derived V-genes.

### Assembly, expression and purification

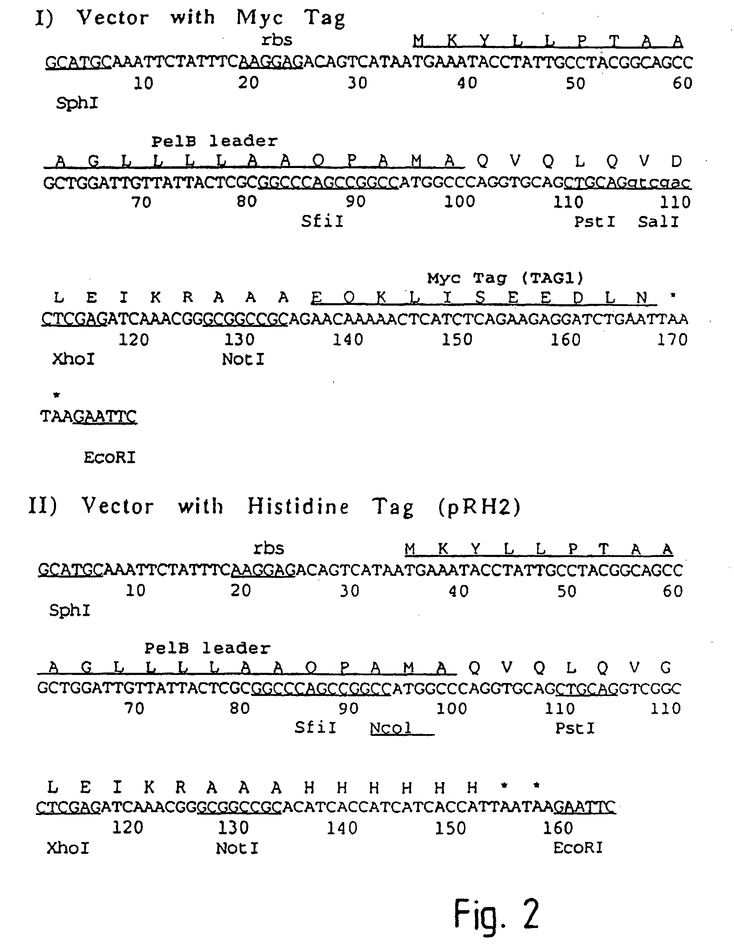

The use of PCR assembly avoids the use of multiple restriction enzymes which may cut V-genes at internal sites. This process used here appears efficient and does not require the separate preparation of a linker fragment (Clackson et al., 1991 Nature 352, 624-628). To check the assembly process the linked product was cloned into an expression vector which included the Myc Tag (Figure 2). Randomly picked clones were grown up and induced as previously described (Hawkins and Winter, 1992). Western Blotting using a monoclonal antibody, 9E10, against the Myc Tag (Ward et al., 1989) demonstrated that 80% of the clones correctly expressed. For ease of purification the scFv frgament was subcloned into the expression vector pRH2 containing a hexahistidine tag. A clone from patient 5 was grown up in a 1 L volume the scFV fragment purified from the periplasm. The yield was estimated as 0.5 mg/L/OD600 based on an A280nm of 1.4 for a 1 mg/ml solution.

### Example 2 - Construction of a Fusion Protein

### Plasmid construction

The BamHI/ClaI env fragment (nt 6537-7674, nt numbering from Shinnick et al, 1981 Nature 293, 543-548) from pCRIP (gift from O. Danos, Danos & Mulligan 1988 PNAS 85, 6460-6464) was cloned into the BamHI/ClaI backbone fragment of pZipNeoSV(X) (gift from R. Mulligan, Cepko et al., 1984 Cell 37, 1053-1062) to generate an intermediate plasmid penvBam/Cla.

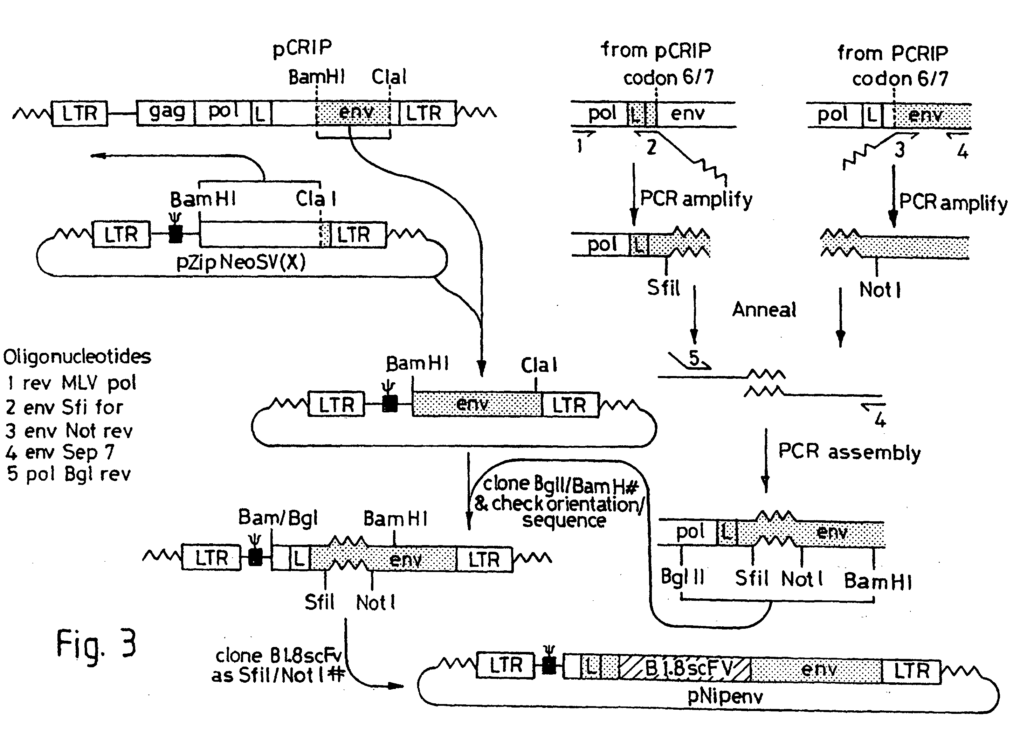

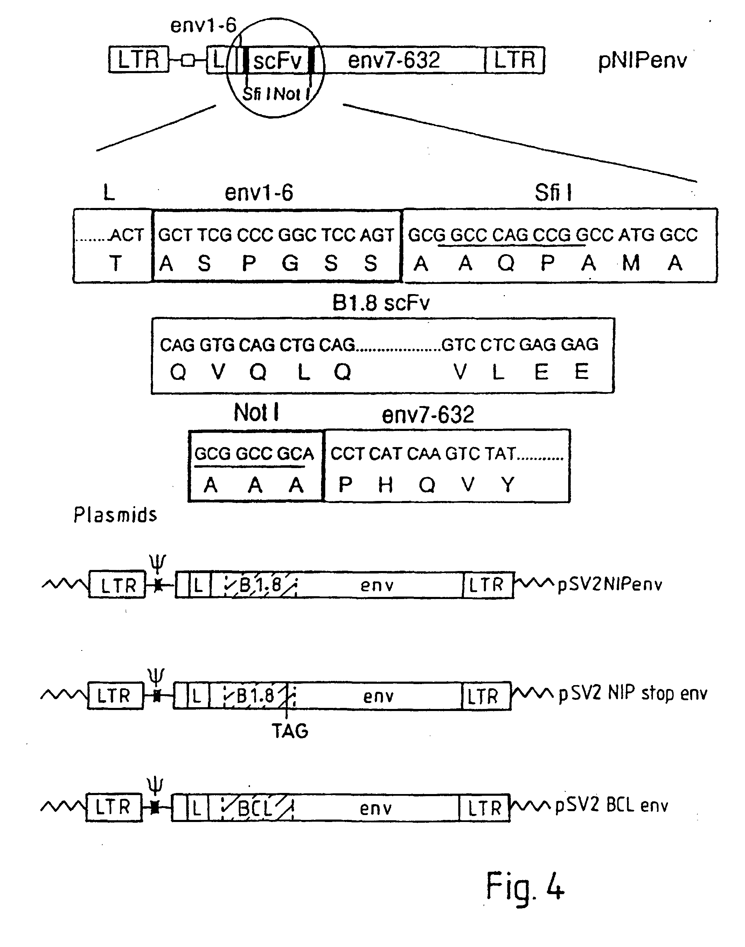

A SfiI/NotI cloning site was introduced beyond the leader peptide sequence between codons corresponding to the 6th and 7th aminoacids (from the N-terminus) in the mature MoMLV env polypeptide. The oligonucleotide pair envNotrev (5'-CTG CAG GAG CTC GAG ATC AAA CGG GCG GCC GCA CCT CAT CAA GTC TAT AAT ATC-3', Seq ID No. 49, complementary to MoMLV env nts 5894-5914 with a 33nt 5' overhang encoding a NotI site and 21nt complementary to the 5' tail of envSfifor) and envseq7 (5'-GCC AGA ACG GGG TTT GGC C-3', Seq ID No. 50, reverse complement of MoMLV env nts 6581-6600) was used to prime amplification (from plasmid pCRIP) of a 739bp fragment downstream of env codon 6. A second oligonucleotide pair, envSfifor (5'-TTT GAT CTC GAG CTC CTG CAG GGC CGG CTG GGC CGC ACT GGA GCC GGG CGA AGC AGT-3', Seq ID No. 51 reverse complement of MoMLV env nts 5873-5893 with a 36nt 5' overhang encoding a SfiI site and 21nt complementary to the 5' tail of envNotrev) and revMLVpol (5'-AAT TAC ATT GTG CAT ACA GAC CC-3', Seq ID No. 52, complementary to MoMLV pol nts 5277-5249) was used to prime amplification (from pCRIP) of a 702bp fragment upstream of env codon 7. Amplifications were carried out using Vent polymerase and reactions were subjected to 15 PCR cycles at 94°C for 1min, 60°C for 1min and 72°C for 1min. The complementary 21nt tails of the 702 and 739bp gel-purified PCR products allowed PCR linkage to generate an env gene fragment incorporating a SfiI/NotI cloning site at the desired position. The two fragments were mixed and subjected to three PCR cycles (94, 40, 72 for 1, 1, and 2 minutes respectively) followed by 17 further cycles of amplification (94°, 60°, 72° for 1, 1 and 2 minutes respectively) after addition of oligonucleotides envseq7 and Bglenvrev (5'-TAA TCA CTA CAG ATC TAG ACT GAC ATG GCG CGT-3', Seq ID No. 53, complementary to MoMLV pol nucleotides 5766 to 5785 with the 5' tail incorporating a BgIII restriction site). The product, a 905bp fragment, was digested with BgIII and BamHI and cloned in forward orientation into the BamHI site of penvBam/Cla (see above) giving the plasmid pSfi/Notenv. Correct assembly of this plasmid was confirmed by restriction analysis and dideoxy sequencing (Sanger et al., 1977 PNAS 74, 5463-5467). A functional B1.8 scFv antibody was then subcloned from prokaryotic expression vector (Hawkins et al., 1992 J. Mol, Biol. 226, 889-896) as an SfiI/NotI fragment into the SfiI/NotI cloning site of pSfi/Not.Env to generate the plasmid pNIP.env (Fig. 3). The sequence across the junctions of pNIPenv is shown in Figure 4 (Seq. ID No. 63 to 66, including translation of nucleotide sequence).

Finally, the modified retroviral envelope expression cassette was subcloned as a HindIII/Eco-RI fragment into a modified pSV2Neo plasmid (a gift from Ashok Venkitaraman, MRC Centre, Cambridge) to generate the plasmid pSVNIPenv (Fig 4).

### Cell transfection

NIH3T3 fibroblasts and the ecotropic retroviral packaging cell line psi2 (Mann et al., 1983 Cell 33, 153-159) were maintained in DMEM/10%FBS supplemented with 60µg/ml benzylpenicillin and 100µg/ml streptomycin at 37°C in atmosphere of 5%CO₂. The cells were replated twice weekly using EDTA without trypsin to disrupt the monolayer.

Plasmid pNIPenv was transfected (with pDCneo, a plasmid containing a neomycin resistance marker) into psi2 cells by calcium phosphate precipitation. Briefly, 2x10⁵ cells were plated in 90mm tissue culture plates (Nunc), cultured overnight, washed and fed with 10mls new medium. 10µl plasmid DNA and 50µl 2M CaCl₂ (0.2µm-filtered) were diluted in sterile water to a volume of 400µl. The CaCl₂/DNA mix was added dropwise to an equal volume of 0.2µm-filtered 2xHEPES-buffered saline (280mM NaCl, 10mM KCl, 1.5mM Na₂HPO₄.2-H₂O, 12mM dextrose, 50mM HEPES, pH adjusted to 7.05 with 0.5N NaOH) and left to stand for 20 minutes at RT. The transfection solution (800ml) was added to the cells which were cultured for 16hrs, washed and refed. G418 selection (1mg/ml) was commenced 24 hrs later and continued for approximately 2 weeks.

Transfected colonies expressing surface B1.8 single chain antibody were identified by panning with NIP.BSA-coated beads. Briefly, Tosyl activated paramagnetic beads (Dynal, Oslo, Norway, Prod. no. 14004) were coated with NIP10.BSA (approximately 10 NIP-caproate-O-succinimide molecules coupled to each bovine serum albumin molecule, Hawkins et al., 1992), washed extensively in PBS and blocked with DMEM/10%FBS. 90mm tissue culture plates containing up to 50 G418-resistant psi2 colonies were rocked gently for 1 hr at 40°C followed by 1 hr at room temperature with 2x10⁷ (50µl) beads in 5mls DMEM/10%FBS. After 5 washes in PBS, positive colonies (heavily coated with paramagnetic beads) were easily identified and were transferred individually for further expansion, cryopreservation and harvest of cell supernatants.

Therefore it was shown that the specificity of the antibody is displayed on the surface of the cells, and therefore that the antibody is folded.

### Construction of a soluble protein expression vector

To create a soluble expression vector a stop codon and frame shift mutation were inserted between the antibody gene and the 3' portion of the MoMLV Envelope gene. The B1.8 scFv fragment was PCR-amplified (10 cycles, 94°C 1 min, 55°C 1 min, 74°C 1 min) using SfiVHBAK (5'-TAC TCG CGG CCC AAC CGG CCA TGG CCC AGG TSM ARC TGC AGS AGT C-3', Seq ID No. 54) and a forward primer Not.STOP (5'- AAC ACT TTC TGC GGC CGC CTC CTC AGA GGA C-3', Seq ID No. 55) encoding the nucleotide insertion to create a stop codon and frameshift 5' of the NotI site. The fragment was then digested with SfiI/NotI and cloned into pSfi/Not.Env to create the plasmid pNIPstop (Fig 4). Plasmid pSVBCLenv (Fig 4) was derived from pSVNIPenv by replacing the B1.8 scFv with a control scFv gene which was PCR-cloned from the BCL1 mouse lymphoma. VH and Vλ genes were PCR cloned and assembled from BCL-derived DNA using standard protocols.

### Preparation of plasmid DNA

Plasmids were amplified in *E. coli* strain TG1 (Gibson, 1984), extracted by alkaline lysis and column purified using the Promega Magic Maxipreps™ DNA purification system (Promega, Madison, WI, USA). The DNA was eluted in water. The purity of the plasmid prep was confirmed by agarose gel electrophoresis and by measuring the A260nm/A280nm ratio (in all cases the ratio was > 1.7). The purified plasmid was stored at -20°C. Prior to use the plasmid was adjusted to 160mg/ml in 200mM NaCl.

### Preparation of B1.8 scFv protein

For bacterial expression, the B1.8 scFv gene was cloned as a PstI/NotI fragment into the vector pRH2, which links a tail of six histidines to the C-terminus of the scFv and was derived by inverse PCR mutagenesis. This plasmid was transformed into *E. coli*, strain TG1 and the scFv protein expressed and purified on an NP-sepharose column as previously described (Hawkins and Winter, 1992 Eur. J. Immunol. 22, 867-870). The purified protein was shown to bind strongly to NIP-BSA using a previously described ELISA (Hawkins and Winter, 1992). As a negative control scFvD1.3 (Hawkins et al., 1992) was cloned into the expression vector pRH2 and expressed and then purified on a lysozyme column as described by Hawkins et al., (1992, cited previously).

### Vaccination protocol

Male BALB/c mice 10 weeks of age were used for immunisation. Pre-immunisation blood samples were obtained by tail bleeds. The blood was centrifuged at 13,000 rpm for 2 minutes in a microcentrifuge to separate the serum. The serum then stored at -20°C for subsequent assay. Two groups of mice were immunised some with DNA and some with protein. The two groups were immunised as described below.
a) Protein vaccine: B1.8 scFv protein was adjusted to a concentration of 250 µg/ml in PBS and mixed with an equal volume of CFA. Mice were challenged subcutaneously with 100ml of this vaccine (12.5µg scFv) at two separate sites. Identical boosts were administered two and four weeks later. 200µl tail bleeds were obtained 10 days after the final boost. Blood samples were processed as above.
b) DNA vaccine: Two groups of three mice were challenged with 50µl (8µg) DNA, either subcutaneously (sc) in both flanks, or by the intramuscular (im) route (right and left quadriceps, total DNA for each mouse 16µg). Two identical booster inoculations were given at one week intervals. 200µl tail bleeds were obtained immediately prior to the first, second and third challenge and one week after the final boost. Serum was separated and stored as above.

### Analysis of immune response

Individual flat-bottomed wells in flexible 96 well assay plates (Falcon 3912 MicroTest III) were coated with B1.8.His or control (D1.3.His anti-lysozyme) scFv protein at 25µg/ml overnight in PBS at room temperature. The use of the histidine tagged scFv to coat the plates has been found to result in more of the protein retaining its antigen binding capacity. Plates were washed x 3 with PBS, blocked for 2 hrs at 37°C with 3% BSA in PBS and washed x 3 in PBS. Test serum was added (diluted 1:100 or 1:1000 in PBS/3%BSA) and incubated for one hour at room temperature. Plates were washed x 3 in PBS and incubated for one hour at room temperature with a second layer HRP-conjugated polyclonal goat anti-mouse Fc antibody at 1:1000 dilution (Sigma, cat. no. A0168). Plates were washed x 4 in PBS, developed with ABTS and the A405nm measured after 30 minutes using a Thermomax™ microplate reader (Molecular devices, Menlo Park, USA).

### Results

### Immune response to protein vaccine

It was first sought to establish whether mice could mount an effective anti-idiotypic humoral immune response when challenged with a scFv murine antibody in CFA. Six mice were challenged subcutaneously with 25µg of the B1.8 anti-NIP scFv in CFA, with booster doses two and four weeks later. Ten days after the final challenge, serum from these animals contained insufficient anti-B1.8 antibody to give a positive ELISA signal at 1:100 dilution of the serum.

### Immune response to DNA vaccine

Plasmid pNIPenv (Fig 3, see materials and methods for details of construction) encodes a chimeric fusion protein consisting of the ecotropic MoMLV envelope polypeptide Pr80env with a scFv anti-NIP antibody fragment (Kd 4x10⁻⁸M) inserted 6 aminoacids from the N-terminus. The 33 aminoacid MoMLV env leader sequence is retained, without disruption of the leader cleavage site. As well as the 6 N-terminal amino-acids from the MoMLV envelope protein the scFv also has a further 6 amino-acids derived from the pelB leader remaining at the N-terminus. Expression is driven from promoter/enhancer sequences in the 5' MoMLV long terminal repeat (LTR). Polyadenylation signal sequences are provided by the 3' MoMLV LTR. When transfected into mouse fibroblasts (described above), it was found that pNIPenv gave stable cell-surface expression of functional B1.8 scFv in fusion with the MoMLVenv protein.

Mice were primed via the subcutaneous (three mice) or intramuscular (three mice) route with 16µg of pSVNIPenv in 200 mM NaCl, with booster doses one and two weeks later.
Control mice were vaccinated pSVBCLenv. Pre-vaccination, pre-booster and one week post-vaccination serum samples were tested by ELISA for a humoral response to B1.8scFv. Prior to the second booster, anti-B1.8 scFv antibodies were detected at 1:100 dilution of the serum in three of the six pSVNIPenv- vaccinated mice, two inoculated im and one sc. One week after the second booster, all six mice had easily detectable anti-B1.8 scFv antibodies which did not crossreact with the D1.3 scFv. Sera from control pBCLenv-vaccinated mice remained negative in the anti-B1.8 ELISA.

### Anamnestic response to protein vaccine after DNA vaccine

After 8 weeks, anti-B1.8 antibody titres had fallen in the pSVNIPenv immunised mice. At this point the three mice, originally inoculated intramuscularly with pNIPenv, were challenged intravenously with 20µg purified B1.8 scFv protein in PBS. Five days later, serum from these mice contained a greatly increased titre of anti-B1.8 antibodies - the average rise was 12 fold and all had antibodies clearly detectable at 1:1000 dilution.

### Boosting with soluble scFv expression vector (pNIPstop)

To test whether boosting with a soluble protein expression vector would also boost the antibody titre, mice were inoculated with pNIPstop. Ten weeks after the primary immunisation, the three mice immunised subcutaneously with pNIPenv were inoculated with 8 µg sc and 8 µg im in 200mM NaCl. Five days after boosting, test bleeds were obtained and assayed for antibody activity. The serum titre increased an average of 10 fold and again all were now positive at 1:1000 dilution.

### Comparison of soluble pNIPstop and pNIPenv in generating primary immune response

To demonstrate the importance of the fusion protein to enhance the immune response we carried out a control experiment to compare the efficacy of two vectors at stimulating a primary immune response. Two groups each consisting of two BALB/c mice were used as before. Serum was obtained by tail bleed as before and then the mice were inoculated weekly x 3 with the appropriate plasmid. Twenty-eight days after the start of immunisation serum was again obtained following tail bleeds and assayed for anti-B1.8 activity. 2/2 from the group inoculated with the pNIPenv plasmid were positive at 1:100 dilution and 2/2 in the pNIPstop group were positive. Clearly the env tag is not necessary to stimulate an immune response.

### Confirmation that immune response recognises the native antigen

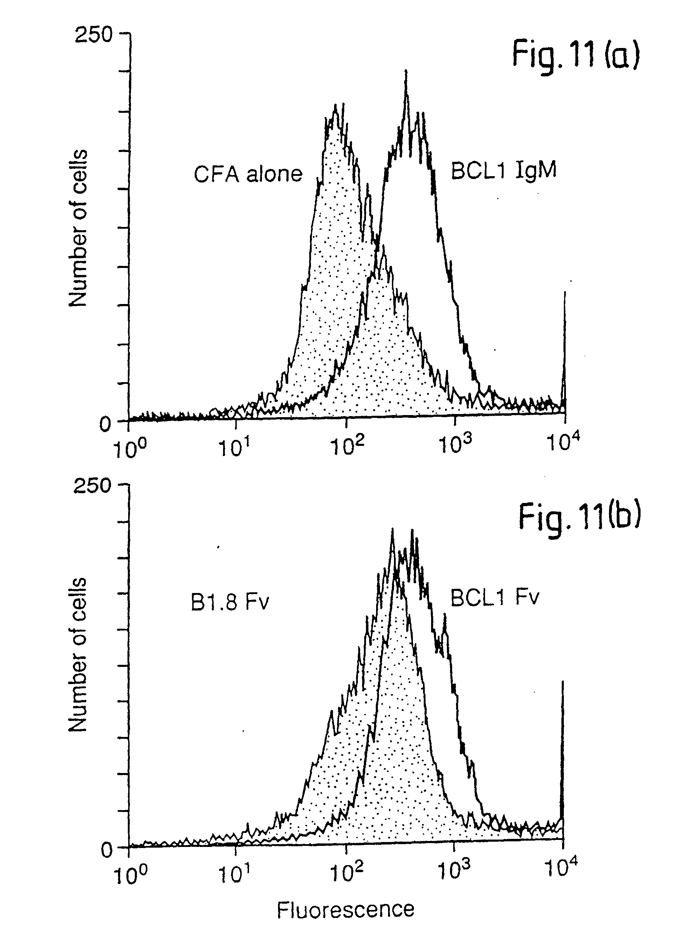

A group of five mice immunised four times with DNA (pSV2-BCL1) encoding the unfused BCL1 scFv fragment also generated humoral responses to the idiotype, as detected by binding to the BCL1 IgM fragment in an ELISA (not shown). Moreover, as a clear demonstration of their ability to recognise native antigen in the form required for therapy, these anti-idiotypic antisera were shown by FACS analysis to bind to lymphoma cells bearing surface BCL1 Ig. BCL1 cells were pre-incubated with serum at a 1:20 dilution before staining with FITC conjugated anti-mouse IgG (Sigma) and followed by FACS analysis. Indeed the immune response was comparable to that for the BCL1 IgM antibody in CFA (Fig. 11). This was in contrast to antisera derived from mice immunised with pSV2-B1.8 which bound only weakly to the BCL1 lymphoma (Fig. 11).

### Example 3 - Construction of a Vector suitable for use in Human Recipients

### Vector Construction

The initial vectors used in example 2 were based on Moloney Murine Leukaemia virus vectors and contained large stretches of unmodified viral sequences (Russell et al., 1993 Nucl. Acids Res. 21, 1081-1085). These vectors were shown to be effective in raising anti-idiotype responses and gave no untoward effects in the mice inoculated. Although there is no evidence of danger to man from such vectors it was decided to modify the vectors to avoid any potential risks. There was some concern about two features of the original vectors: the retroviral envelope gene (as it could theoretically be recombined into another retrovirus thus changing its tropism) and the packaging signal (which might allow packaging of the injected DNA into existing human retroviruses). Whilst changing the vector it was decided to incorporate changes which improve the vectors for use in man:- the promoter used to drive expression of the idiotypic scFv was changed to the Rous Sarcoma Virus (RSV) promoter as that has been shown to give expression when directly injected into non-human primate muscle (Jiao et al., 1992 Hum. Gene Ther. 3, 21-33). The present inventors also used a vector which contains a bacterial single strand origin of replication to allow the production of ssDNA which will facilitate sequencing the scFv portion (which is specific for that individual patient) of the vector before injecting into the patient. The vector used is based on a commercially available vector pRc/RSV (British Biotechnology/Invitrogen).

To convert this vector backbone into a vector suitable for genetic immunisation it was desirable to introduce leader sequences, termination signals and to allow for the production of fusion proteins. Fusion proteins do not appear to be necessary for the production of anti-idiotype responses but one way of enhancing the immune response might be to attach suitable proteins - perhaps foreign proteins or perhaps cytokines (Tao & Levy, 1993 Nature 362, 755-758). As fusion proteins were not necessary in animal models the initial human trial will use only a short peptide tag but this is one area for potential future modifications to the protocol.

The vector pSfi/Not.Tag1 was modified to replace the pelB leader with the human immunoglobulin VH1 leader sequence which permits the encoding of an Sfi I cloning site without modification of the amino-acid sequence. This was introduced with oligonucleotides using the HindIII/Pst I cloning sites and confirmed by sequencing.

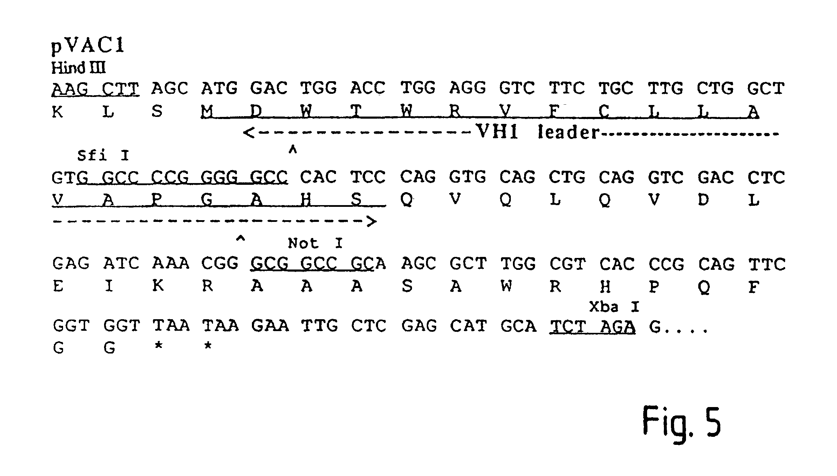

This was then cloned as an EcoRI/Blunt - HindIII fragment into the Not I/Blunt - HindIII cut vector pRc/RSV to give the sequence ( Seq ID No. 56) between the HindIII/XbaI sites as shown in Figure 5. The scFv for an individual patient can be inserted at the sites shown by the symbol ^.

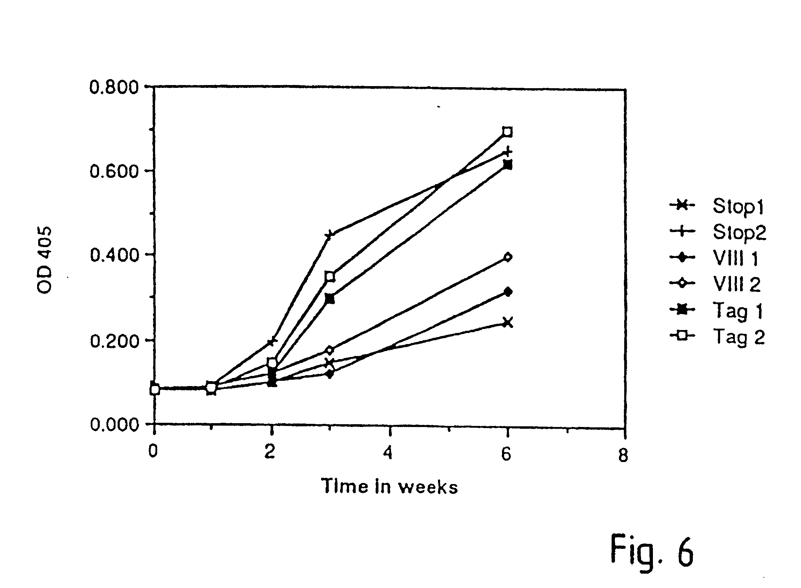

The vector was then tested in two ways:
(i) The scFv B1.8 was cloned into the vector and then the resultant construct transfected into two cell lines - NSO (a myeloma cell line) and NIH 3T3 (a fibroblast cell line). Utilising the neomycin resistance gene in pRc/RSV stable transfectants were isolated and the supernatant assayed for scFv B1.8 antigen binding activity. In both cases the antibody fragment was expressed and bound to the hapten NIP - the antigen recognised by the monoclonal antibody B1.8. Clones were isolated from the NSO transfected cells and shown to produce 1-3 mg/L of functional scFv in spent culture supernatant.
(ii) The plasmid was used in a genetic immunisation experiment and compared to pSV2-B1.8. They gave comparable results and appear to be superior to a further vector which encodes the Fd bacteriophage gene 8 protein between the Not I and Xba I site (Figure 6). The likely explanation is that in transfection experiments the level of expression was 10-100 fold lower for the scFv B1.8-Gene 8 fusion. Other investigators have found a strong correlation of level of expression and immune response when using genetic immunisation to raise immune responses to viral proteins (G. Rhodes, personal communication). It thus appears that short peptide tags are sufficient to raise an anti-idiotypic immune response and we thus propose to use such a peptide tag in our trials. The tag will also be useful to test for expression in cell culture. The tags used to date are either virus derived or human derived and it is not clear that they are essential and certainly there is no difference between them. To avoid any possible dangers we will use a synthetic tag derived to bind streptavidin as this could also be useful to detect expression in vitro (Schmidt & Skerra, 1993 Protein Engineering 6, 109-122). As stated above this is one feature that can be readily modified to include other proteins such as cytokines if necessary.

Figure 6 (Immunisation of mice with vectors utilising the RSV promoter) shows the results of idiotypic immunisation against the scFv B1.8. The response for individual mice as determined by ELISA at 1:100 serum dilution are shown. The mice were immunised intra muscularly at weeks 0, 1, 2. Note one mouse with the stop vector (pSV2 - B1.8) had a poor response and the response of mice immunised with the gene 8 fusion vector were poor (VIII 1 and VIII2) whereas both those with the peptide tag vector gave good responses (Tag1 and Tag2).

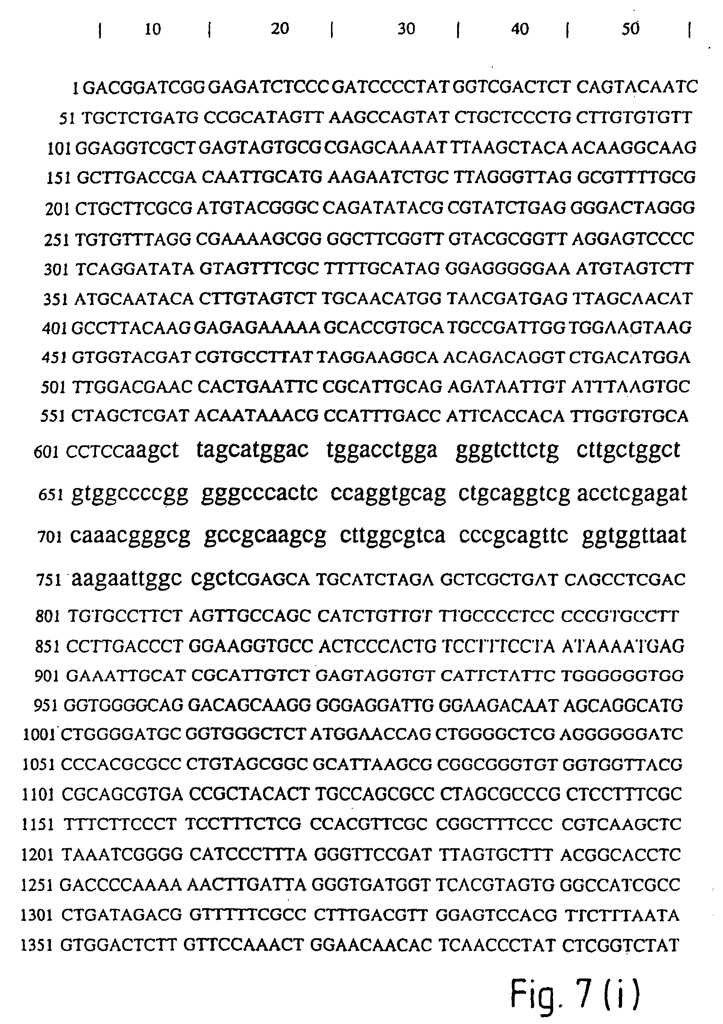

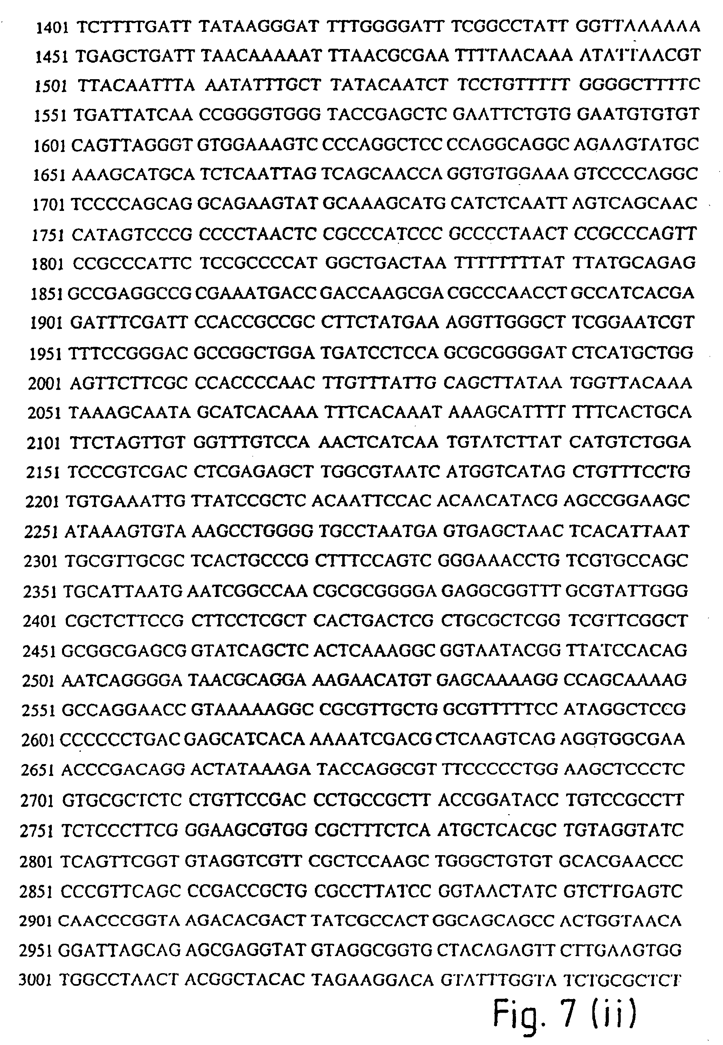

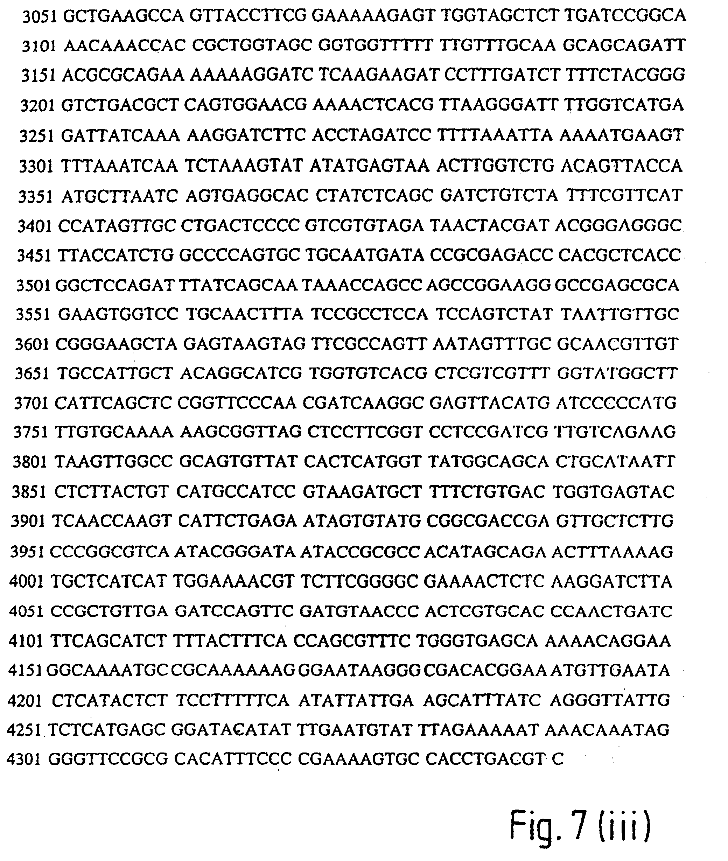

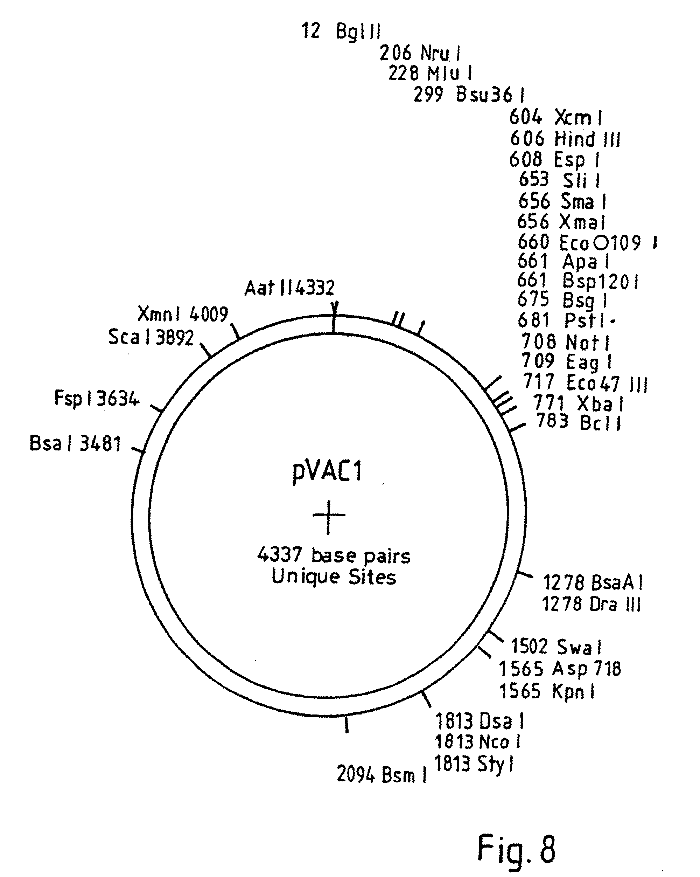

The sequence (Seq ID No. 58) of the final vector pVACI is given in Figure 7 together with a map of the unique restriction sites (Figure 8). The sequence in lower case letters in Figure 7 corresponds to the sequence shown in Figure 5 through to the two stop codons. The vector pVAC1 is available from the present inventors (at the Cambridge Centre for Protein Engineering, Cambridge, United Kingdom).

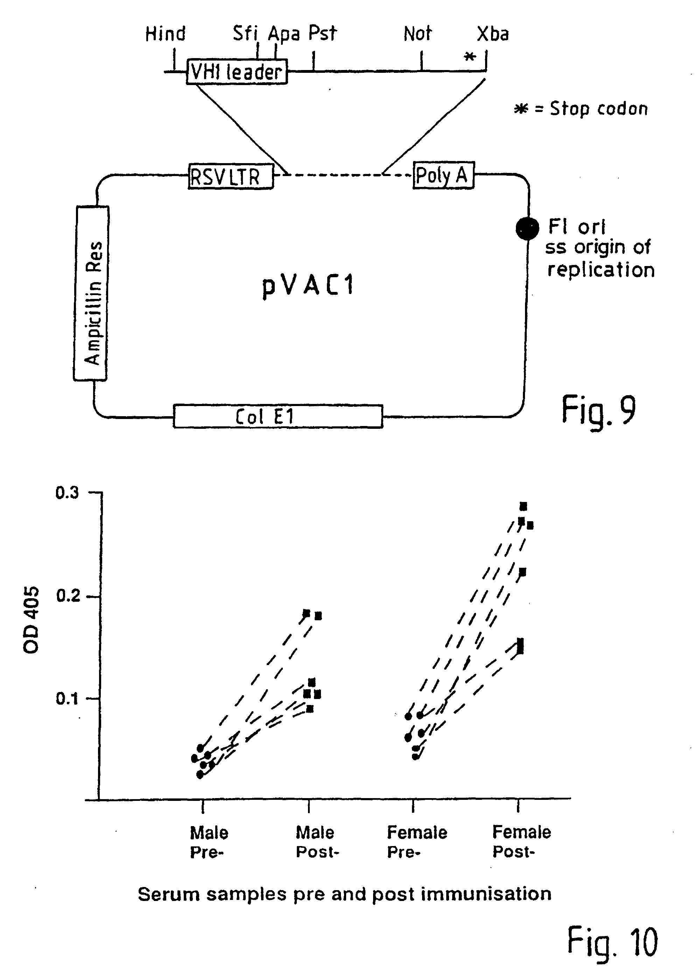

Figure 9 shows a diagrammatic representation of the vector pVAC1 indicating important restriction sites and important genes.

Figure 10 is a graph of O.D. (405nm) against time for male and female mice immunised (by direct injection of DNA) with the pVAC vector expressing B1.8scfv (pVAC1.B1-8). The graph shows that, for individual mice, there was a clear increase in titre following immunisation.

Although the neomycin resistance gene is useful for in vitro testing it is unnecessary for human immunisation. The SV40 promoter used to drive the neomycin resistance gene is also associated with the same risks as other strong promoters. The plasmid to be used for human trials thus has the neomycin gene deleted by an SfiI/Bst BI digest and then blunt ligation. This prevents any such risk.

### Discussion

The present inventors have demonstrated that a plasmid vaccine encoding a single chain murine antibody/retroviral envelope fusion protein induces a strong humoral immune response to the antibody moiety in BALB/c mice, whereas vaccination with the purified scFv protein mixed with Complete Freunds Adjuvant gives no detectable response. Induction of B-cell memory appears to occur as boosting with either soluble protein or a soluble scFv expression vector was effective at producing a rapid rise in antibody titre.

The humoral immune response to protein encoded by the plasmid vaccine implies that cellular uptake and expression of the plasmid must have occurred in vivo. Expression of genes inoculated directly into muscle has been demonstrated previously (Wolff et al., 1990 cited previously) but to our knowledge, this is the first evidence for an immune response to a self or altered self polypeptide following direct injection of naked DNA. Cellular uptake and expression of genes is increased when the DNA is coated onto gold particles and fired into the tissues by a particle gun (Tang et al., 1992, cited previously). More involved methods of gene transfer include the use of viral vectors, encapsulating the DNA into liposomes, coupling of DNA to cationic liposomes or to the outside of viruses (for review see Miller, 1992 Nature 357, 45-46). These have the advantage of increased efficiency of transfer but, by comparison with direct injection of purified plasmid DNA, these alternative approaches are somewhat involved and raise more safety issues.

The humoral anti-B1.8 response to the plasmid vaccine pNIPenv was clearly superior to that raised against purified B1.8 scFv protein mixed with Complete Freunds Adjuvant. The approach disclosed herein has several advantages. Following gene transfer, there is likely to be a continuous supply of the target antigen, diminishing over a period of days or weeks, whereas injected protein may have a very short half-life. Using the plasmid vaccine the B1.8 scFv is at least partially anchored to the cell surface, probably incorporated into oligomeric env protein structures. The association between the two components of MoMLV env (p15TM and gp70SU) is stabilised largely by hydrophobic interactions and gp70SU can dissociate from p15TM. Following gene transfer, the fusion protein will therefore be displayed to the immune system as oligomeric, polyvalent (multiple copies per cell) cell-associated antigen or as soluble antigen. This prolonged exposure to newly synthesised antigen may be important for an optimal immune response and this argument has been used to explain the superiority of live compared to killed viral vaccines.

Antigen-specific T helper cells can amplify both humoral and cellular immune responses by direct cell interaction and by providing appropriate stimulatory cytokines. Several mechanisms can be envisaged whereby the plasmid vaccine may recruit helper T cells more efficiently, but one deserves special mention. Antigen-specific T helper cell activation and proliferation are triggered by MHC class-II associated peptides displayed at the surface of B lymphocytes, macrophages and other antigen presenting cells. The MHC class-II associated peptides are derived from ingested antigen. B cells specifically recognising epitopes on B1.8 scFv will internalise and process the antigen to generate peptides for surface display. With the B1.8 scFv fused to a viral envelope protein, any B1.8 specific B cells will have more scope for generation of peptides (many of which will be derived from the highly immunogenic env protein) which can recruit helper T cells. A concern with such an approach is that T-cell memory to the weakly immunogenic B1.8 may not develop, resulting in a weak or absent anamnestic immune humoral immune response. This, however, was not a problem as both soluble, scB1.8 Fv protein given intravenously and the plasmid, pNIPstop, soluble expression vector produced a rapid rise in antibody titre.

Cytotoxic T-cells may be important in cell mediated immunity to tumours and viruses (reviewed by Greenburg and Riddell, 1992 J. Natl. Cancer Inst. 84, 1059-1061) and here again there are potentially important differences between the protein-based and plasmid vaccines used in this study. As well as expressing the fusion protein in various forms (see above), the MHC class-I positive transduced cells will also display a range of B1.8 scFv and/or env-derived MHC class-I associated peptides recognisable by cytotoxic T lymphocytes. This should help to stimulate an effective antigen specific cytotoxic T-cell response.

Regardless of the mechanisms involved, the vaccination strategy employed in this study gave a strong humoral immune response to a weakly immunogenic single chain antibody fragment and was superior to vaccination with purified protein plus adjuvant. The scFv gene used in this study could be replaced with a variety of genes or gene fragments encoding weakly immunogenic self or altered self proteins. Initial appropriate target antigens for such therapy are patient-specific vaccines against unique surface immunoglobulins and T cell receptors expressed by certain B cell and T cell malignancies. Rapid PCR cloning and assembly of antibody VH and VL genes from surface immunoglobulin positive human B cell lymphoma tissue has already been demonstrated. Single chain T-cell receptors have also been cloned and expressed (Soo Hoo et al., 1992 PNAS 89, 4759-4763) and so, in principle, such an approach could also be applied to T-cell malignancies. Extension to non-malignant disease may be possible if it is confirmed that T-cell, and perhaps B-cell, receptor usage is restricted in auto-immune diseases such as multiple sclerosis and rheumatoid arthritis (for review see Marguerie et al., 1992 Immunol. Today 13, 336-338). It may then be possible, using in-cell PCR assembly (Embleton et al., 1992 Nucl. Acids Res. 20, 3831-3837) to identify oligoclonal use of B/T cell receptors and thus to generate appropriate vaccines. The approach could also be useful for the rapid generation of strain-specific vaccines against highly mutable viruses such as HIV-1 and influenza virus.

The generation of cytotoxic T-cell response to the antigen should theoretically be possible with this approach and is the subject of continuing investigation. If this proves to be possible then further uses may be envisaged. Increasingly, growth regulatory proteins are being found in mutated forms or as fusion proteins (resulting from translocations) in malignant cells (for review see Urban and Schreiber, 1992 Ann. Rev. Immunol. 10, 617-644). Such abnormal intracellular proteins are potential tumour specific targets for cytotoxic T-cells (for review see Greenburg and Riddell, 1992 cited above). Frequently a spectrum of changes are found, often with more than one oncogene being affected in any individual tumour. Thus, PCR cloning of the mutant genes and the use of vector such as that described here may be a practical method of generating tumour specific T-cell immunity on an individual patient basis.

There are possible improvements to the constructs described here. Fusion to the MoMLV env protein was used in this study, but it is likely that other immunogenic proteins could be substituted as fusion partners. Coat proteins of other enveloped viruses and immunogenic cell surface or secreted proteins derived from any pathogenic organism or non human species may be suitable alternatives. Indeed it may be valuable to use several antigenic fusion partners to help prevent the theoretical problem that the immune response the highly immunogenic moiety of the fusion protein could ultimately overwhelm any response to the real (weakly immunogenic) target antigen.

One advantage of the genetic approach to vaccination is that it potentially allows efficient use of the natural method of presenting antigen, which should therefore engage a wide range of effector systems. Moreover, manipulation and improvement of the response obtained should be relatively easy; for example, it may be possible to improve the efficiency of presentation of antigen to T cells by expressing molecules with co-stimulatory activity together with the immunogen. One important molecule involved in co-stimulation is B7, which interacts with CD28 expressed by T cells thereby providing accessory signals for T-cell activation (Galvin et al., 1992 J. Immunol. 12, 3802-3808). New vectors will be constructed which express both B7 and the idiotypic Ig. Sequences of both mouse and human B7 are published (Freeman et al., 1989 J. Immunol. 8, 2714-2722) and the genes will be cloned by PCR. An expression cassette will then be inserted into our vectors which express idiotypic sequences.

A number of cytokines are known to improve aspects of antigen presentation and the direct delivery of expression vectors containing cytokine genes could enhance vaccine efficacy. Interferon gamma is one example which could be useful due to the property of upregulating MHC expression (Gaczynska et al. 1993 Nature 365, 264-267). The gene could be encoded on a separate vector and the amount varied independently.

Episomal vectors such as those based on EBV or papova virus may have advantages over current vectors. These should allow high copy number episomal replication and may be more effective. New vectors utilising the pVAC1 HindIII/Xbal insert have been constructed with the expression plasmid pCEP4 (Invitrogen) which contains the EBV origin of replication and EBNA. These have given enhanced levels of expression and greater stability of expression in cell culture experiments using the human osteosarcoma line 791T and may be more efficient in vivo although they also raise new safety issues for human use. More efficient methods of transfection such as liposome mediated and receptor mediated delivery may also improve the efficiency of the process.

The flexibility and simplicity of the direct DNA delivery will facilitate the rapid testing of a variety of strategies as suggested above. Combined with continuing progress in understanding antigen presentation, this should enhance our ability to generate active antitumour immunity. This approach is clearly very flexible and may be useful for immunising with numbers of other tumour antigens. These might include the human papilloma virus E6/E7 proteins involved in cervical carcinoma; mutant p53/ras involved in many cancers and possibly oncofoetal antigens such as carcinoembryonic antigen. In addition the approach outlined here may be useful for viruses such as HIV which has a high rate of mutation in vivo leading to significant variation in encoded antigens between individuals.

In summary, the present invention provides a simple, effective, vaccine strategy for immunisation with weakly immunogenic self or altered self proteins. In specific embodiments, PCR amplification (and assembly) of the target gene is followed by restriction and cloning of the PCR product into the appropriately digested expression plasmid. A large scale preparation of the resulting plasmid DNA is then used directly for inoculation. The expression plasmid can produce a disease marker in fusion, or in conjunction, with other immunomodulatory polypeptides to modulate the immune response to achieve a therapeutic goal. The need for costly in vitro protein expression and purification systems is bypassed and will facilitate the development of vaccines for a variety of diseases, especially those for which they must be tailor made for individuals or subgroups of patients.

In the foregoing, sequence ID Nos. 49-66 are shown as sequence ID Nos. 1-18 in the second sequence listing below.

### SEQUENCE LISTING

(1) GENERAL INFORMATION:
   (i) APPLICANT:
      (A) NAME: Medical Research Council
      (B) STREET: 20 Park Crescent
      (C) CITY: London
      (E) COUNTRY: United Kingdom
      (F) POSTAL CODE (ZIP) : W1N 4AL
      (G) TELEPHONE: (071) 636 5422
      (H) TELEFAX: (071) 323 1331

      (A) NAME: Hawkins, Robert Edward
      (B) STREET: 6 The Lawns
      (C) CITY: Cambridge
      (E) COUNTRY: United Kingdom
      (F) POSTAL CODE (ZIP): CB3 ORU

      (A) NAME: Russell, Stephen James
      (B) STREET: 10 Courtyards, Little Shelford
      (C) CITY: Cambridge
      (E) COUNTRY: United Kingdom
      (F) POSTAL CODE (ZIP): CB2 5ER

      (A) NAME: Stevenson, Freda Katherine
      (B) STREET: 9 Meadowhead Road, Bassett
      (C) CITY: Southampton
      (E) COUNTRY: United Kingdom
      (F) POSTAL CODE (ZIP) : SO1 7AD

      (A) NAME: Winter, Gregory Paul
      (B) STREET: 64 Cavendish Avenue
      (C) CITY: Cambridge
      (E) COUNTRY: United Kingdom
      (F) POSTAL CODE (ZIP): CB1 4UT
   (ii) TITLE OF INVENTION: Improvements in or relating to
      immune response modification
   (iii) NUMBER OF SEQUENCES: 66
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.25 (EPO)
(2) INFORMATION FOR SEQ ID NO:1:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:1:
   CAGGTGCAGC TGGTGCAGTC TG 22
(2) INFORMATION FOR SEQ ID NO:2:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:2:
   CAGGTCAACT TAAGGGAGTC TG 22
(2) INFORMATION FOR SEQ ID NO:3:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:3:
   GAGGTGCAGC TGGTGGAGTC TG 22
(2) INFORMATION FOR SEQ ID NO:4:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:4:
   CAGGTGCAGC TGCAGGAGTC GG 22
(2) INFORMATION FOR SEQ ID NO:5:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:5:
   GAGGTGCAGC TGCTGCAGTC TG 22
(2) INFORMATION FOR SEQ ID NO:6:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:6:
   CAGGTACAGC TGCAGCAGTC AG 22
(2) INFORMATION FOR SEQ ID NO:7:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:7:
   TGAGGAGACG GTGACCAGGG TGCC 24
(2) INFORMATION FOR SEQ ID NO:8:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:8:
   TGAGGAGACG GTGACCAGGG TTCC 24
(2) INFORMATION FOR SEQ ID NO:9:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:9:
   TGAAGAGACG GTGACCATTG TCCC 24
(2) INFORMATION FOR SEQ ID NO:10:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:10:
   TGAGGAGACG GTGACCGTGG TCCC 24
(2) INFORMATION FOR SEQ ID NO:11:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:11:
   GACATCCAGA TGACCCAGTC TCC 23
(2) INFORMATION FOR SEQ ID NO:12:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:12:
   GATATTGTGA TGACTCAGTC TCC 23
(2) INFORMATION FOR SEQ ID NO:13:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:13:
   GAAATTGTGT TGACGCAGTC TCC 23
(2) INFORMATION FOR SEQ ID NO:14:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:14:
   GAAACGACAC TCACGCAGTC TCC 23
(2) INFORMATION FOR SEQ ID NO:15:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:15:
   ACGTTTGATC TCCACCTTGG TCCC 24
(2) INFORMATION FOR SEQ ID NO:16:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:16:
   ACGTTTGATA TCCACTTTGG TCCC 24
(2) INFORMATION FOR SEQ ID NO:17:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:17:
   ACGTTTAATC TCCAGTCGTG TCCC 24
(2) INFORMATION FOR SEQ ID NO:18:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:18:
   CAGTCTGTGT TGACGCAGCC GCCCTC 26
(2) INFORMATION FOR SEQ ID NO:19:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:19:
   CAGTCTGCCC TGACTCAGCC TGCCTC 26
(2) INFORMATION FOR SEQ ID NO:20:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:20:
   TCCTATGAGC TGACTCAGCC ACVCTC 26
(2) INFORMATION FOR SEQ ID NO:21:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:21:
   CACGTTATAC TGACTCAACC GCCCTC 26
(2) INFORMATION FOR SEQ ID NO:22:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:22:
   ACCTAGGACG GTGACCTTGG TCCC 24
(2) INFORMATION FOR SEQ ID NO:23:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:23:
   ACCTAGGACG GTCAGCTTGG TCCC 24
(2) INFORMATION FOR SEQ ID NO:24:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:24:
   ACCGAGGACG GTCAGCTGGG TGCC 24
(2) INFORMATION FOR SEQ ID NO:25:
   (i) SEQUENCE CHARACTERISTICS:
      (A) LENGTH: 41 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:25:
   TCGCGGCCCA ACCGGCCATG GCCCAGGTGC AGCTGGTGCA G 41
(2) INFORMATION FOR SEQ ID NO:26:
   (i) SEQUENCE CHARACTERISTICS:
      (A) LENGTH: 41 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:26:
   TCGCGGCCCA ACCGGCCATG GCCCAGGTCA ACTTAAGGGA G 41
(2) INFORMATION FOR SEQ ID NO:27:
   (i) SEQUENCE CHARACTERISTICS:
      (A) LENGTH: 41 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:27:
   TCGCGGCCCA ACCGGCCATG GCCGAGGTGC AGCTGGTGGA G 41
(2) INFORMATION FOR SEQ ID NO:28:
   (i) SEQUENCE CHARACTERISTICS:
      (A) LENGTH: 41 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:28:
   TCGCGGCCCA ACCGGCCATG GCCCAGGTGC AGCTGCAGGA G 41
(2) INFORMATION FOR SEQ ID NO:29:
   (i) SEQUENCE CHARACTERISTICS:
      (A) LENGTH: 41 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:29:
   TCGCGGCCCA ACCGGCCATG GCCGAGGTGC AGCTGCTGCA G 41
(2) INFORMATION FOR SEQ ID NO:30:
   (i) SEQUENCE CHARACTERISTICS:
      (A) LENGTH: 41 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:30:
   TCGCGGCCCA ACCGGCCATG GCCCAGGTAC AGCTGCAGCA G 41
(2) INFORMATION FOR SEQ ID NO:31:
   (i) SEQUENCE CHARACTERISTICS:
      (A) LENGTH: 50 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:31:
   AGAGCCACCT CCGCCTGAAC CGCCTCCACC TGAGGAGACG GTGACCAGGG 50
(2) INFORMATION FOR SEQ ID NO:32:
   (i) SEQUENCE CHARACTERISTICS:
      (A) LENGTH: 50 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:32:
   AGAGCCACCT CCGCCTGAAC CGCCTCCACC TGAGGAGACG GTGACCAGGG   50
(2) INFORMATION FOR SEQ ID NO:33:
   (i) SEQUENCE CHARACTERISTICS:
      (A) LENGTH: 50 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:33:
   AGAGCCACCT CCGCCTGAAC CGCCTCCACC TGAAGAGACG GTGACCATTG 50
(2) INFORMATION FOR SEQ ID NO:34:
   (i) SEQUENCE CHARACTERISTICS:
      (A) LENGTH: 50 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:34:
   AGAGCCACCT CCGCCTGAAC CGCCTCCACC TGAGGAGACG GTGACCGTGG 50
(2) INFORMATION FOR SEQ ID NO:35:
   (i) SEQUENCE CHARACTERISTICS:
      (A) LENGTH: 48 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:35:
   GGCGGAGGTG GCTCTGGCGG TGGCGGATCG GACATCCAGA TGACCCAG 48
(2) INFORMATION FOR SEQ ID NO:36:
   (i) SEQUENCE CHARACTERISTICS:
      (A) LENGTH: 49 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:36:
   GGCGGAGGTG GCTCTGGCGG TGGCGGATCG GATATTGTGA TGACTCAGT 49
(2) INFORMATION FOR SEQ ID NO:37:
   (i) SEQUENCE CHARACTERISTICS:
      (A) LENGTH: 49 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:37:
   GGCGGAGGTG GCTCTGGCGG TGGCGGATCG GAAATTGTGT TGACGCAGT 49
(2) INFORMATION FOR SEQ ID NO:38:
   (i) SEQUENCE CHARACTERISTICS:
      (A) LENGTH: 48 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:38:
   GGCGGAGGTG GCTCTGGCGG TGGCGGATCG GAAACGACAC TCACGCAG 48
(2) INFORMATION FOR SEQ ID NO:39:
   (i) SEQUENCE CHARACTERISTICS:
      (A) LENGTH: 41 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:39:
   GATATGAGAT ACTGCGGCCG CACGTTTGAT CTCCACCTTG G 41
(2) INFORMATION FOR SEQ ID NO:40:
   (i) SEQUENCE CHARACTERISTICS:
      (A) LENGTH: 41 base pairs
      (B) TYPE: nucleic acid
      (C) STRANDEDNESS: single
      (D) TOPOLOGY: linear
   (ii) MOLECULE TYPE : DNA (genomic)
   (iii) HYPOTHETICAL: NO
   (iii) ANTI-SENSE: NO
   (xi) SEQUENCE DESCRIPTION: SEQ ID NO:40:
   GATATGAGAT ACTGCGGCCG CACGTTTGAT ATCCACTTTG G 41
(2) INFORMATION FOR SEQ ID NO:41:
   (i) SEQUENCE CHARACTERISTICS:
      (A) LENGTH: 41 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:41:
   GATATGAGAT ACTGCGGCCG CACGTTTAAT CTCCAGTCGT G 41
(2) INFORMATION FOR SEQ ID NO:42:
   (i) SEQUENCE CHARACTERISTICS:
      (A) LENGTH: 48 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:42:
   GGCGGAGGTG GCTCTGGCGG TGGCGGATCG CAGTCTGTGT TGACGCAG 48
(2) INFORMATION FOR SEQ ID NO:43:
   (i) SEQUENCE CHARACTERISTICS:
      (A) LENGTH: 48 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:43:
   GGCGGAGGTG GCTCTGGCGG TGGCGGATCG CAGTCTGCCC TGACTCAG 48
(2) INFORMATION FOR SEQ ID NO:44:
   (i) SEQUENCE CHARACTERISTICS:
      (A) LENGTH: 48 base pairs
      (B) TYPE: nucleic acid
      (C) STRANDEDNESS: single
      (D) TOPOLOGY: linear
   (ii) MOLECULE TYPE : DNA (genomic)
   (iii) HYPOTHETICAL: NO
   (iii) ANTI-SENSE: NO
   (xi) SEQUENCE DESCRIPTION: SEQ ID NO:44:
   GGCGGAGGTG GCTCTGGCGG TGGCGGATCG TCCTATGAGC TGACTCAG 48
(2) INFORMATION FOR SEQ ID NO:45:
   (i) SEQUENCE CHARACTERISTICS:
      (A) LENGTH: 49 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:45:
   GGCGGAGGTG GCTCTGGCGG TGGCGGATCG CACGTTATAC TGACTCAAC 49
(2) INFORMATION FOR SEQ ID NO:46:
   (i) SEQUENCE CHARACTERISTICS:
      (A) LENGTH: 41 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:46:
   GATATGAGAT ACTGCGGCCG CACCTAGGAC GGTGACCTTG G 41
(2) INFORMATION FOR SEQ ID NO:47:
   (i) SEQUENCE CHARACTERISTICS:
      (A) LENGTH: 41 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:47:
   GATATGAGAT ACTGCGGCCG CACCTAGGAC GGTCAGCTTG G 41
(2) INFORMATION FOR SEQ ID NO:48:
   (i) SEQUENCE CHARACTERISTICS:
      (A) LENGTH: 41 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:48:
   GATATGAGAT ACTGCGGCCG CACCGAGGAC GGTCAGCTGG G 41
(2) INFORMATION FOR SEQ ID NO:49:
   (i) SEQUENCE CHARACTERISTICS:
      (A) LENGTH: 54 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:49:
(2) INFORMATION FOR SEQ ID NO:50:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:50:
   GCCAGAACGG GGTTTGGCC 19
(2) INFORMATION FOR SEQ ID NO:51:
   (i) SEQUENCE CHARACTERISTICS:
      (A) LENGTH: 57 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:51:
(2) INFORMATION FOR SEQ ID NO:52:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:52:
   AATTACATTG TGCATACAGA CCC 23
(2) INFORMATION FOR SEQ ID NO:53:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:53:
   TAATCACTAC AGATCTAGAC TGACATGGCG CGT 33
2) INFORMATION FOR SEQ ID NO:54:
   (i) SEQUENCE CHARACTERISTICS:
      (A) LENGTH: 46 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:54:
   TACTCGCGGC CCAACCGGCC ATGGCCCAGG TSMARCTGCA GSAGTC 46
(2) INFORMATION FOR SEQ ID NO:55:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:55:
   AACAGTTTCT GCGGCCGCCT CCTCAGAGGA C 31
(2) INFORMATION FOR SEQ ID NO:56:
   (i) SEQUENCE CHARACTERISTICS:
      (A) LENGTH: 172 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:56:
   AAGCTTAGCA TGGACTGGAC CTGGAGGGTC TTCTGCTTGC TGGCTGTGGC      50
   CCCGGGGGCC CACTCCCAGG TGCAGCTGCA GGTCGACCTC GAGATCAAAC     100
   GGGCGGCCGC AAGCGCTTGG CGTCACCCGC AGTTCGGTGG TTAATAAGAA     150
   TTGCTCGAGC ATGCATCTAG AG                                   172
(2) INFORMATION FOR SEQ ID NO:57:
   (i) SEQUENCE CHARACTERISTICS:
      (A) LENGTH: 47 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iii) ANTI-SENSE: NO
   (v) FRAGMENT TYPE: internal
   (xi) SEQUENCE DESCRIPTION: SEQ ID NO:57:
   Lys Leu Ser Met Asp Trp Thr Trp Arg Val Phe Cys Leu Leu
   1               5                   10
   Ala Val Ala Pro Gly Ala His Ser Gln Val Gln Leu Gln Val
   15                  20                  25
   Asp Leu Glu Ile Lys Arg Ala Ala Ala Ser Ala Trp Arg His
       30                  35                  40
   Pro Gln Phe Gly Gly
           45
(2) INFORMATION FOR SEQ ID NO:58:
   (i) SEQUENCE CHARACTERISTICS:
      (A) LENGTH: 4341 base pairs
      (B) TYPE: nucleic acid
      (C) STRANDEDNESS: single
      (D) TOPOLOGY: circular
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:58:
   GACGGATCGG GAGATCTCCC GATCCCCTAT GGTCGACTCT CAGTACAATC   50
   TGCTCTGATG CCGCATAGTT AAGCCAGTAT CTGCTCCCTG CTTGTGTGTT  100
   GGAGGTCGCT GAGTAGTGCG CGAGCAAAAT TTAAGCTACA ACAAGGCAAG  150
   GCTTGACCGA CAATTGCATG AAGAATCTGC TTAGGGTTAG GCGTTTTGCG  200
   CTGCTTCGCG ATGTACGGGC CAGATATACG CGTATCTGAG GGGACTAGGG  250
   TGTGTTTAGG CGAAAAGCGG GGCTTCGGTT GTACGCGGTT AGGAGTCCCC  300
   TCAGGATATA GTAGTTTCGC TTTTGCATAG GGAGGGGGAA ATGTAGTCTT  350
   ATGCAATACA CTTGTAGTCT TGCAACATGG TAACGATGAG TTAGCAACAT  400
   GCCTTACAAG GAGAGAAAAA GCACCGTGCA TGCCGATTGG TGGAAGTAAG  450
   GTGGTACGAT CGTGCCTTAT TAGGAAGGCA ACAGACAGGT CTGACATGGA  500
   TTGGACGAAC CACTGAATTC CGCATTGCAG AGATAATTGT ATTTAAGTGC  550
   CTAGCTCGAT ACAATAAACG CCATTTGACC ATTCACCACA TTGGTGTGCA  600
   CCTCCAAGCT TAGCATGGAC TGGACCTGGA GGGTCTTCTG CTTGCTGGCT  650
   GTGGCCCCGG GGGCCCACTC CCAGGTGCAG CTGCAGGTCG ACCTCGAGAT  700
   CAAACGGGCG GCCGCAAGCG CTTGGCGTCA CCCGCAGTTC GGTGGTTAAT  750
   AAGAATTGGC CGCTCGAGCA TGCATCTAGA GCTCGCTGAT CAGCCTCGAC  800
   TGTGCCTTCT AGTTGCCAGC CATCTGTTGT TTGCCCCTCC CCCGTGCCTT  850
   CCTTGACCCT GGAAGGTGCC ACTCCCACTG TCCTTTCCTA ATAAAATGAG  900
   GAAATTGCAT CGCATTGTCT GAGTAGGTGT CATTCTATTC TGGGGGGTGG  950
   GGTGGGGCAG GACAGCAAGG GGGAGGATTG GGAAGACAAT AGCAGGCATG 1000
   CTGGGGATGC GGTGGGCTCT ATGGAACCAG CTGGGGCTCG AGGGGGGATC 1050
   CCCACGCGCC CTGTAGCGGC GCATTAAGCG CGGCGGGTGT GGTGGTTACG 1100
   CGCAGCGTGA CCGCTACACT TGCCAGCGCC CTAGCGCCCG CTCCTTTCGC 1150
   TTTCTTCCCT TCCTTTCTCG CCACGTTCGC CGGCTTTCCC CGTCAAGCTC 1200
   TAAATCGGGG CATCCCTTTA GGGTTCCGAT TTAGTGCTTT ACGGCACCTC 1250
   GACCCCAAAA AACTTGATTA GGGTGATGGT TCACGTAGTG GGCCATCGCC 1300
   CTGATAGACG GTTTTTCGCC CTTTGACGTT GGAGTCCACG TTCTTTAATA 1350
   GTGGACTCTT GTTCCAAACT GGAACAACAC TCAACCCTAT CTCGGTCTAT 1400
   TCTTTTGATT TATAAGGGAT TTTGGGGATT TCGGCCTATT GGTTAAAAAA 1450
   TGAGCTGATT TAACAAAAAT TTAACGCGAA TTTTAACAAA ATATTAACGT 1500
   TTACAATTTA AATATTTGCT TATACAATCT TCCTGTTTTT GGGGCTTTTC 1550
   TGATTATCAA CCGGGGTGGG TACCGAGCTC GAATTCTGTG GAATGTGTGT 1600
   CAGTTAGGGT GTGGAAAGTC CCCAGGCTCC CCAGGCAGGC AGAAGTATGC 1650
   AAAGCATGCA TCTCAATTAG TCAGCAACCA GGTGTGGAAA GTCCCCAGGC 1700
   TCCCCAGCAG GCAGAAGTAT GCAAAGCATG CATCTCAATT AGTCAGCAAC 1750
   CATAGTCCCG CCCCTAACTC CGCCCATCCC GCCCCTAACT CCGCCCAGTT 1800
   CCGCCCATTC TCCGCCCCAT GGCTGACTAA TTTTTTTTAT TTATGCAGAG 1850
   GCCGAGGCCG CGAAATGACC GACCAAGCGA CGCCCAACCT GCCATCACGA 1900
   GATTTCGATT CCACCGCCGC CTTCTATGAA AGGTTGGGCT TCGGAATCGT 1950
   TTTCCGGGAC GCCGGCTGGA TGATCCTCCA GCGCGGGGAT CTCATGCTGG 2000
   AGTTCTTCGC CCACCCCAAC TTGTTTATTG CAGCTTATAA TGGTTACAAA 2050
   TAAAGCAATA GCATCACAAA TTTCACAAAT AAAGCATTTT TTTCACTGCA 2100
   TTCTAGTTGT GGTTTGTCCA AACTCATCAA TGTATCTTAT CATGTCTGGA 2150
   TCCCGTCGAC CTCGAGAGCT TGGCGTAATC ATGGTCATAG CTGTTTCCTG 2200
   TGTGAAATTG TTATCCGCTC ACAATTCCAC ACAACATACG AGCCGGAAGC 2250
   ATAAAGTGTA AAGCCTGGGG TGCCTAATGA GTGAGCTAAC TCACATTAAT 2300
   TGCGTTGCGC TCACTGCCCG CTTTCCAGTC GGGAAACCTG TCGTGCCAGC 2350
   TGCATTAATG AATCGGCCAA CGCGCGGGGA GAGGCGGTTT GCGTATTGGG    2400
   CGCTCTTCCG CTTCCTCGCT CACTGACTCG CTGCGCTCGG TCGTTCGGCT    2450
   GCGGCGAGCG GTATCAGCTC ACTCAAAGGC GGTAATACGG TTATCCACAG    2500
   AATCAGGGGA TAACGCAGGA AAGAACATGT GAGCAAAAGG CCAGCAAAAG    2550
   GCCAGGAACC GTAAAAAGGC CGCGTTGCTG GCGTTTTTCC ATAGGCTCCG    2600
   CCCCCCTGAC GAGCATCACA AAAATCGACG CTCAAGTCAG AGGTGGCGAA    2650
   ACCCGACAGG ACTATAAAGA TACCAGGCGT TTCCCCCTGG AAGCTCCCTC    2700
   GTGCGCTCTC CTGTTCCGAC CCTGCCGCTT ACCGGATACC TGTCCGCCTT    2750
   TCTCCCTTCG GGAAGCGTGG CGCTTTCTCA ATGCTCACGC TGTAGGTATC    2800
   TCAGTTCGGT GTAGGTCGTT CGCTCCAAGC TGGGCTGTGT GCACGAACCC    2850
   CCCGTTCAGC CCGACCGCTG CGCCTTATCC GGTAACTATC GTCTTGAGTC    2900
   CAACCCGGTA AGACACGACT TATCGCCACT GGCAGCAGCC ACTGGTAACA    2950
   GGATTAGCAG AGCGAGGTAT GTAGGCGGTG CTACAGAGTT CTTGAAGTGG    3000
   TGGCCTAACT ACGGCTACAC TAGAAGGACA GTATTTGGTA TCTGCGCTCT    3050
   GCTGAAGCCA GTTACCTTCG GAAAAAGAGT TGGTAGCTCT TGATCCGGCA    3100
   AACAAACCAC CGCTGGTAGC GGTGGTTTTT TTGTTTGCAA GCAGCAGATT    3150
   ACGCGCAGAA AAAAAGGATC TCAAGAAGAT CCTTTGATCT TTTCTACGGG    3200
   GTCTGACGCT CAGTGGAACG AAAACTCACG TTAAGGGATT TTGGTCATGA    3250
   GATTATCAAA AAGGATCTTC ACCTAGATCC TTTTAAATTA AAAATGAAGT    3300
   TTTAAATCAA TCTAAAGTAT ATATGAGTAA ACTTGGTCTG ACAGTTACCA    3350
   ATGCTTAATC AGTGAGGCAC CTATCTCAGC GATCTGTCTA TTTCGTTCAT    3400
   CCATAGTTGC CTGACTCCCC GTCGTGTAGA TAACTACGAT ACGGGAGGGC    3450
   TTACCATCTG GCCCCAGTGC TGCAATGATA CCGCGAGACC CACGCTCACC    3500
   GGCTCCAGAT TTATCAGCAA TAAACCAGCC AGCCGGAAGG GCCGAGCGCA    3550
   GAAGTGGTCC TGCAACTTTA TCCGCCTCCA TCCAGTCTAT TAATTGTTGC    3600
   CGGGAAGCTA GAGTAAGTAG TTCGCCAGTT AATAGTTTGC GCAACGTTGT    3650
   TGCCATTGCT ACAGGCATCG TGGTGTCACG CTCGTCGTTT GGTATGGCTT    3700
   CATTCAGCTC CGGTTCCCAA CGATCAAGGC GAGTTACATG ATCCCCCATG    3750
   TTGTGCAAAA AAGCGGTTAG CTCCTTCGGT CCTCCGATCG TTGTCAGAAG    3800
   TAAGTTGGCC GCAGTGTTAT CACTCATGGT TATGGCAGCA CTGCATAATT    3850
   CTCTTACTGT CATGCCATCC GTAAGATGCT TTTCTGTGAC TGGTGAGTAC    3900
   TCAACCAAGT CATTCTGAGA ATAGTGTATG CGGCGACCGA GTTGCTCTTG    3950
   CCCGGCGTCA ATACGGGATA ATACCGCGCC ACATAGCAGA ACTTTAAAAG    4000
   TGCTCATCAT TGGAAAACGT TCTTCGGGGC GAAAACTCTC AAGGATCTTA    4050
   CCGCTGTTGA GATCCAGTTC GATGTAACCC ACTCGTGCAC CCAACTGATC    4100
   TTCAGCATCT TTTACTTTCA CCAGCGTTTC TGGGTGAGCA AAAACAGGAA    4150
   GGCAAAATGC CGCAAAAAAG GGAATAAGGG CGACACGGAA ATGTTGAATA    4200
   CTCATACTCT TCCTTTTTCA ATATTATTGA AGCATTTATC AGGGTTATTG    4250
   TCTCATGAGC GGATACATAT TTGAATGTAT TTAGAAAAAT AAACAAATAG    4300
   GGGTTCCGCG CACATTTCCC CGAAAAGTGC CACCTGACGT C             4341
(2) INFORMATION FOR SEQ ID NO:59:
   (i) SEQUENCE CHARACTERISTICS:
      (A) LENGTH: 189 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:59:
   GCATGCAAAT TCTATTTCAA GGAGACAGTC ATAATGAAAT ACCTATTGCC      50
   TACGGCAGCC GCTGGATTGT TATTACTCGC GGCCCAGCCG GCCATGGCCC     100
   AGGTGCAGCT GCAGGTCGAC CTCGAGATCA AACGGGCGGC CGCAGAACAA     150
   AAACTCATCT CAGAAGAGGA TCTGAATTAA TAAGAATTC                 189
(2) INFORMATION FOR SEQ ID NO:60:
   (i) SEQUENCE CHARACTERISTICS:
      (A) LENGTH: 48 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iii) ANTI-SENSE: NO
   (v) FRAGMENT TYPE : internal
   (xi) SEQUENCE DESCRIPTION: SEQ ID NO:60:
   Met Lys Tyr Leu Leu Pro Thr Ala Ala Ala Gly Leu Leu Leu
   1               5                   10
   Leu Ala Ala Gln Pro Ala Met Ala Gln Val Gln Leu Gln Val
   15                  20                  25
   Asp Leu Glu Ile Lys Arg Ala Ala Ala Glu Gln Lys Leu Ile
       30                  35                  40
   Ser Glu Glu Asp Leu Asn
           45
(2) INFORMATION FOR SEQ ID NO:61:
   (i) SEQUENCE CHARACTERISTICS:
      (A) LENGTH: 174 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:61:
   GCATGCAAAT TCTATTTCAA GGAGACAGTC ATAATGAAAT ACCTATTGCC      50
   TACGGCAGCC GCTGGATTGT TATTACTCGC GGCCCAGCCG GCCATGGCCC     100
   AGGTGCAGCT GCAGGTCGGC CTCGAGATCA AACGGGCGGC CGCACATCAC     150
   CATCATCACC ATTAATAAGA ATTC                                 174
(2) INFORMATION FOR SEQ ID NO:62:
   (i) SEQUENCE CHARACTERISTICS:
      (A) LENGTH: 43 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iii) ANTI-SENSE: NO
   (v) FRAGMENT TYPE: internal
   (xi) SEQUENCE DESCRIPTION: SEQ ID NO:62:
   Met Lys Tyr Leu Leu Pro Thr Ala Ala Ala Gly Leu Leu Leu
   1               5                   10
   Leu Ala Ala Gln Pro Ala Met Ala Gln Val Gln Leu Gln Val
   15                  20                  25
   Gly Leu Glu Ile Lys Arg Ala Ala Ala His His His His His
       30                  35                  40
   His
(2) INFORMATION FOR SEQ ID NO:63:
   (i) SEQUENCE CHARACTERISTICS:
      (A) LENGTH: 57 base pairs
      (B) TYPE: nucleic acid
      ( C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:63:
   ACTGCTTCGC CCGGCTCCAG TGCGGCCCAG CCGGCCATGG CCCAGGTGCA 50
   GCTGCAG 57
(2) INFORMATION FOR SEQ ID NO:64:
   (i) SEQUENCE CHARACTERISTICS:
      (A) LENGTH: 19 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iii) ANTI-SENSE: NO
   (v) FRAGMENT TYPE: internal
   (xi) SEQUENCE DESCRIPTION: SEQ ID NO:64:
   Thr Ala Ser Pro Gly Ser Ser Ala Ala Gln Pro Ala Met Ala
   1 5 10
   Gln Val Gln Leu Gln
   15
(2) INFORMATION FOR SEQ ID NO:65:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO:65:
   GTCCTCGAGG AGGCGGCCGC ACCTCATCAA GTCTAT 36
(2) INFORMATION FOR SEQ ID NO:66:
   (i) SEQUENCE CHARACTERISTICS:
      (A) LENGTH: 12 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iii) ANTI-SENSE: NO
   (v) FRAGMENT TYPE: internal
   (xi) SEQUENCE DESCRIPTION: SEQ ID NO:66:

**Table 2:**

| **RESULTS OF SEQUENCE ANALYSIS FROM PATIENT SPECIMENS.** | | | | |
|---|---|---|---|---|
| | **IDENTICAL SEQUENCES (NUMBER SEQUENCED)** | | | |
| | **VH** | **Vλ** | **Vk** | **% HISTOLOGICAL TUMOUR INVOLVEMENT** |
| **Controls:** | | | | |
| NLN | 0(12) | 0(8) | 0(8) | |
| | | | | |
| SPL | 0(10) | 0(10) | 0(10) | |

| **Patients:** | | | | |
|---|---|---|---|---|
| 1 | 9(16)* | 7(16)* | ND | 60 |
| | | | | |
| 2 | 4(18)* | 4(16)* | ND | 90 |
| | | | | |
| 3 | 3(10) | ND | 2(10) | 50 |
| | | | | |
| 4 | 3(18)* | -** | ND | 60⁺ |
| | | | | |
| 5 | 5(12) | ND | 5(9) | 64 |

| | | | | |
|---|---|---|---|---|
| Notes: * indicates sequences confirmed by sequencing of the V-genes of a heterohybridoma. | | | | |
| ** the light chain PCR from this patient failed. | | | | |
| + Sample used was peripheral blood. NLN - normal lymph node, SPL-spleen, ND not done. | | | | |

## Claims

1. An immunomodulatory composition to modulate the immune response against a tumour associated antigen, said composition comprising a nucleic acid sequence directing the expression of said tumour associated antigen in a form which interacts with the immune system of the individual, wherein said tumour associated antigen is selected from an idiotypic determinant expressed on the surface of a B or T-cell malignancy, an oncogene product or an oncofoetal antigen.

2. An immunomodulatory composition according to claim 1, wherein the tumour associated antigen is an idiotypic determinant expressed on the surface of a B or T cell malignancy.

3. An immunomodulatory composition according to claim 1 or claim 2, wherein said nucleic acid is in an unencapsidated form.

4. An immunomodulatory composition according to claim 1 or claim 2, further comprising a second nucleic acid sequence directing the expression of a further immunomodulatory polypeptide for the purpose of further modulating the immune response of the individual to the tumour associated antigen.

5. An immunomodulatory composition according to claim 4, wherein said second nucleic acid sequence is comprised on the same nucleic acid molecule as the nucleic acid sequence of claim 1.

6. An immunomodulatory composition according to claim 4 or claim 5, wherein said tumour associated antigen and said further immunomodulatory polypeptide are co-expressed or expressed as a fusion peptide in the individual.

7. An immunomodulatory composition according to any one of claims 4 to 6, wherein the said further immunomodulatory polypeptide comprises a cytokine or a foreign amino acid sequence.

8. An immunomodulatory composition according to any one of claims 4 to 7, wherein said nucleic acid sequence or second nucleic acid sequence comprises a sequence which causes said tumour associated antigen, and/or said further immunomodulatory polypeptide, to become membrane associated.

9. An immunomodulatory composition according to any one of claims 4 to 8, wherein said nucleic acid sequence and/or said second nucleic acid sequence comprise DNA.

10. A vector comprising the nucleotide sequence of nucleotides 606 to 716 of the sequence shown in Figure 7.

11. A vector according to claim 10, comprising the nucleotide sequence of nucleotides 606 to 780 of the sequence shown in Figure 7.

12. A vector comprising the nucleotide sequence of nucleotides 775 to 1 (in the 5' to 3' direction) of the sequence shown in Figure 7.

13. A vector according to any one of claims 10, 11 or 12, comprising the nucleotide sequence of pVAC1, shown in Figure 7.

14. A method of making an immunomodulatory composition according to claim 1, comprising the steps of identifying the nucleic acid sequence encoding the tumour associated antigen by analysis of a suitable sample from a patient; extracellularly cloning said tumour associated antigen therefrom; and inserting said nucleic acid sequence into a suitable vector.

15. A method according to claim 14, wherein the nucleic acid sequence encoding said tumour associated antigen is cloned from a patient be means of PCR.

16. Use of an immunomodulatory composition according to claim 1, in the preparation of a medicament for modifying the immune response of an individual, wherein said modification comprise delivery into living cells in vivo of the nucleic acid sequence directing expression of said tumour associated antigen in a form which interacts with the immune system of the individual.

17. Use according to claim 16, wherein said nucleic acid sequence is extracellularly cloned from samples obtained from said individual.

18. Use according to claims 16 or 17, wherein the nucleic acid is delivered in unencapsidated form.

19. Use according to any one of claims 16 to 18 further comprising delivery of the second nucleic acid sequence to the individual to direct the expression of a further immunomodulatory polypeptide for the purpose of further modulating the immune response of the individual to tumour associated antigen.

20. Use according to claim 19, wherein said second nucleic acid sequence is comprised on the same nucleic acid molecule as the nucleic acid sequence of claim 16.

21. Use according to claim 19 or 20, wherein said tumour associated antigen and said further immunomodulatory polypeptide are co-expressed or expressed as a fusion polypeptide in the individual.

22. Use according to any one of claims 19, 20 or 21, wherein said further immunomodulatory polypeptide comprises a cytokine or a foreign amino acid sequence.

23. Use according to any one of claims 16 to 22, wherein said nucleic acid sequence or second said nucleic acid sequence comprises a sequence which causes said tumour associated antigen, and/or said further immunomodulatory polypeptide, to become membrane associated.

24. Use according to any one of claims 16 to 23, wherein said nucleic acid sequence and/or said second nucleic acid sequence comprise DNA.

25. Use according to any one of claims 16 to 24, wherein the tumour associated antigen comprises: an idiotypic determinant from a B cell surface-displayed immunoglobulin molecule; an idiotypic determinant from a T cell receptor; an oncogene product; or an oncofoetal antigen.

26. Use according to any one of claims 16 to 25, wherein the immune response of an individual to a tumour associated antigen is enhanced.

## Patentansprüche

1. Immunmodulationszusammensetzung, um die Immunreaktion auf ein Tumor-assoziiertes Antigen zu modulieren, wobei die Zusammensetzung eine Nucleinsäuresequenz umfasst, die die Expression des Tumor-assoziierten Antigens in eine Form lenkt, die mit dem Immunsystem des Individuums wechselwirkt, worin das Tumor-assoziierte Antigen aus einer idiotypischen Determinante, die auf der Oberfläche einer B-oder T-Zellen-Malignität exprimiert wird, einem Onkogenprodukt oder einem onkofötalen Antigen ausgewählt ist.

2. Immunmodulationszusammensetzung nach Anspruch 1, worin das Tumor-assoziierte Antigen eine idiotypische Determinante ist, die auf der Oberfläche einer B- oder T-Zellen-Malignität exprimiert wird.

3. Immunmodulationszusammensetzung nach Anspruch 1 oder 2, worin die Nucleinsäure in nichtcapsidierter Form vorliegt.

4. Immunmodulationszusammensetzung nach Anspruch 1 oder 2, die weiters eine zweite Nucleinsäuresequenz umfasst, die die Expression eines weiteren Immunmodulationspolypeptids lenkt, um die Immunreaktion des Individuums auf das Tumor-assoziierte Antigen weiter zu modulieren.

5. Immunmodulationszusammensetzung nach Anspruch 4, worin die zweite Nucleinsäuresequenz auf demselben Nucleinsäuremolekül vorliegt wie die Nucleinsäuresequenz aus Anspruch 1.

6. Immunmodulationszusammensetzung nach Anspruch 4 oder 5, worin das Tumor-assoziierte Antigen und das weitere Immunmodulationspolypeptid im Individuum coexprimiert oder als Fusionspeptid exprimiert werden.

7. Immunmodulationszusammensetzung nach einem der Ansprüche 4 bis 6, worin das weitere Immunmodulationspolypeptid ein Cytokin oder eine fremde Aminosäuresequenz umfasst.

8. Immunmodulationszusammensetzung nach einem der Ansprüche 4 bis 7, worin die Nucleinsäuresequenz oder die zweite Nucleinsäuresequenz eine Sequenz umfasst, die bewirkt, dass das Tumor-assoziierte Antigen und/oder das weitere Immunmodulationspolypeptid Membran-assoziiert werden.

9. Immunmodulationszusammensetzung nach einem der Ansprüche 4 bis 8, worin die Nucleinsäuresequenz und/oder die zweite Nucleinsäuresequenz DNA umfassen.

10. Vektor, der eine Nucleotidsequenz aus den Nucleotiden 606 bis 716 der in Fig. 7 gezeigten Sequenz umfasst.

11. Vektor nach Anspruch 10, der eine Nucleotidsequenz aus den Nucleotiden 606 bis 780 der in Fig. 7 gezeigten Sequenz umfasst.

12. Vektor, der eine Nucleotidsequenz aus den Nucleotiden 775 bis 1 (in 5'→3'-Richtung) der in Fig. 7 gezeigten Sequenz umfasst.

13. Vektor nach einem der Ansprüche 10, 11 oder 12, der die in Fig. 7 gezeigte Nucleotidsequenz von pVAC1 umfasst.

14. Verfahren zur Herstellung einer Immunmodulationszusammensetzung gemäß Anspruch 1, umfassend die Schritte des Identifizierens der Nucleinsäuresequenz, die für das Tumor-assoziierte Antigen kodiert, durch Analyse einer geeigneten Probe von einem Patienten; des davon ausgehenden extrazellulären Klonierens des Tumor-assoziierten Antigens; und des Insertierens der Nucleinsäuresequenz in einen geeigneten Vektor.

15. Verfahren nach Anspruch 14, worin die Nucleinsäuresequenz, die für das Tumor-assoziierte Antigen kodiert, mittels PCR von einem Patienten kloniert wird.

16. Verwendung einer Immunmodulationszusammensetzung nach Anspruch 1 bei der Herstellung eines Medikaments zum Modifizieren der Immunreaktion eines Individuums, worin die Modifikation die Abgabe der Nucleinsäuresequenz, die die Expression des Tumor-assoziierten Antigens in eine Form lenkt, die mit dem Immunsystem des Individuums wechselwirkt, in lebende Zellen in vivo umfasst.

17. Verwendung nach Anspruch 16, worin die Nucleinsäuresequenz extrazellulär ausgehend von vom Individuum erhaltenen Proben kloniert wird.

18. Verwendung nach Anspruch 16 oder 17, worin die Nucleinsäure in nichtcapsidierter Form abgegeben wird.

19. Verwendung nach einem der Ansprüche 16 bis 18, die weiters die Abgabe der zweiten Nucleinsäuresequenz an das Individuum umfasst, um die Expression eines weiteren Immunmodulationspolypeptids zu lenken, um die Immunreaktion des Individuums auf Tumor-assoziiertes Antigen weiter zu modulieren.

20. Verwendung nach Anspruch 19, worin die zweite Nucleinsäuresequenz auf demselben Nucleinsäuremolekül vorliegt wie die Nucleinsäuresequenz aus Anspruch 16.

21. Verwendung nach Anspruch 19 oder 20, worin das Tumor-assoziierte Antigen und das weitere Immunmodulationspolypeptid im Individuum coexprimiert oder als Fusionspolypeptid exprimiert werden.

22. Verwendung nach einem der Ansprüche 19, 20 oder 21, worin das weitere Immunmodulationspolypeptid ein Cytokin oder eine fremde Aminosäuresequenz umfasst.

23. Verwendung nach einem der Ansprüche 16 bis 22, worin die Nucleinsäuresequenz oder die zweite Nucleinsäuresequenz eine Sequenz umfasst, die bewirkt, dass das Tumor-assoziierte Antigen und/oder das weitere Immunmodulationspolypeptid Membran-assoziiert werden.

24. Verwendung nach einem der Ansprüche 16 bis 23, worin die Nucleinsäuresequenz und/oder die zweite Nucleinsäuresequenz DNA umfassen.

25. Verwendung nach einem der Ansprüche 16 bis 24, worin das Tumor-assoziierte Antigen Folgendes umfasst: eine idiotypische Determinante eines auf B-Zellen-Oberflächen präsentierten Immunglobulinmoleküls; eine idiotypische Determinante eines T-Zellen-Rezeptors; ein Onkogen-Produkt; oder ein onkofötales Antigen.

26. Verwendung nach einem der Ansprüche 16 bis 25, worin die Immunreaktion eines Individuums auf ein Tumor-assoziiertes Antigen verstärkt wird.

## Revendications

1. Composition immunomodulatrice pour moduler la réponse immune contre un antigène associé à une tumeur, ladite composition comprenant une séquence d'acide nucléique dirigeant l'expression dudit antigène associé à la tumeur sous une forme qui interagit avec le système immun de l'individu, où ledit antigène associé à la tumeur est sélectionné parmi un déterminant idiotypique exprimé sur la surface d'une malignité des cellules B ou T, un produit oncogène ou un antigène oncofoetal.

2. Composition immunomodulatrice selon la revendication 1, où l'antigène associé à une tumeur est un déterminant idiotypique exprimé à la surface d'une malignité d'une cellule B ou T.

3. Composition immunomodulatrice selon la revendication 1 ou la revendication 2, où ledit acide nucléique est sous une forme non encapsidée.

4. Composition immunomodulatrice selon la revendication 1 ou la revendication 2, comprenant de plus une seconde séquence d'acide nucléique dirigeant l'expression d'un autre polypeptide immunomodulateur dans le but de moduler encore la réponse immune de l'individu à l'antigène associé à la tumeur.

5. Composition immunomodulatrice selon la revendication 4, où ladite seconde séquence d'acide nucléique est comprise sur la même molécule d'acide nucléique que la séquence d'acide nucléique de la revendication 1.

6. Composition immunomodulatrice selon la revendication 4 ou la revendication 5, où ledit antigène associé à une tumeur et ledit autre polypeptide immunomodulateur sont co-exprimés ou exprimés en tant que peptide de fusion chez l'individu.

7. Composition immunomodulatrice selon l'une quelconque des revendications 4 à 6, où ledit autre polypeptide immunomodulateur comprend une cytokine ou une séquence étrangère d'acides aminés.

8. Composition immunomodulatrice selon l'une quelconque des revendications 4 à 7, où ladite séquence d'acide nucléique ou seconde séquence d'acide nucléique comprend une séquence qui force ledit antigène associé à la tumeur et/ou ledit autre polypeptide immunomodulateur à se trouver associé à la membrane.

9. Composition immunomodulatrice selon l'une quelconque des revendications 4 à 8, où ladite séquence d'acide nucléique et/ou ladite seconde séquence d'acide nucléique comprend un ADN.

10. Vecteur comprenant la séquence de nucléotides des nucléotides 606 à 716 de la séquence montrée à la Figure 7.

11. Vecteur selon la revendication 10 comprenant la séquence de nucléotides des nucléotides 606 à 780 de la séquence montrée à la Figure 7.

12. Vecteur comprenant la séquence de nucléotides des nucléotides 775 à 1 (dans la direction 5' à 3') de la séquence montrée à la Figure 7.

13. Vecteur selon l'une quelconque des revendications 10, 11 ou 12 comprenant la séquence de nucléotides de Pvacl, montrée à la Figure 7.

14. Méthode de production d'une composition immunomodulatrice selon la revendication 1, comprenant les étapes d'identifier la séquence d'acide nucléique codant pour l'antigène associé à la tumeur par analyse d'un échantillon approprié d'un patient ; le clonage extracellulaire dudit antigène associé à la tumeur à partir de cela ; et l'insertion de ladite séquence d'acide nucléique dans un vecteur approprié.

15. Méthode selon la revendication 14, où la séquence d'acide nucléique codant pour ledit antigène associé à la tumeur est cloné à partir d'un patient au moyen de PCR.

16. Utilisation d'une composition immunomodulatrice selon la revendication 1, dans la préparation d'un médicament pour modifier la réponse immune d'un individu, où ladite modification comprend la délivrance, dans des cellules vivantes in vivo, de la séquence d'acide nucléique dirigeant l'expression dudit antigène associé à la tumeur sous une forme qui interagit avec le système immun de l'individu.

17. Utilisation selon la revendication 16, où ladite séquence d'acide nucléique est extracellulairement clonée à partir d'échantillons obtenus dudit individu.

18. Utilisation selon la revendication 16 ou 17, où l'acide nucléique est délivré sous une forme non encapsidée.

19. Utilisation selon l'une quelconque des revendications 16 à 18, comprenant de plus la délivrance de ladite seconde séquence d'acide nucléique à l'individu pour diriger l'expression d'un autre polypeptide immunomodulateur afin de mieux moduler la réponse immune de l'individu à l'antigène associé à la tumeur.

20. Utilisation selon la revendication 19, où ladite seconde séquence d'acide nucléique se compose de la même molécule d'acide nucléique que la séquence d'acide nucléique de la revendication 16.

21. Utilisation selon la revendication 19 ou 20, où ledit antigène associé à la tumeur et ledit autre polypeptide immunomodulateur sont co-exprimés ou exprimés en tant que polypeptide de fusion chez l'individu.

22. Utilisation selon l'une quelconque des revendications 19, 20 ou 21, où ledit autre polypeptide immunomodulateur comprend une cytokine ou une séquence étrangère d'acides aminés.

23. Utilisation selon l'une quelconque des revendications 16 à 22, où ladite séquence d'acide nucléique ou ladite seconde séquence d'acide nucléique comprend une séquence qui force l'antigène associé à la tumeur et/ou ledit autre polypeptide immunomodulateur, à se trouver associé à la membrane.

24. Utilisation selon l'une quelconque des revendications 16 à 23, où ladite séquence d'acide nucléique et/ou ladite seconde séquence d'acide nucléique comprennent un ADN.

25. Utilisation selon l'une quelconque des revendications 16 à 24, où l'antigène associé à la tumeur comprend : un déterminant idiotypique d'une molécule d'immunoglobine présentée sur une surface d'une cellule B ; un déterminant idiotypique d'un récepteur de cellule T ; un produit oncogène ; ou un antigène oncofoetal.

26. Utilisation selon l'une quelconque des revendications 16 à 25, où la réponse immune d'un individu à un antigène associé à une tumeur est améliorée.
